# EUROPEAN PATENT APPLICATION

(11) **EP 2 279 727 A2**
(43) Date of publication of application: **02.02.2011**
(21) Application number: 10012426.2
(22) Date of filing: 13.09.2006
(51) Int. Cl.: A61K 9/14, A61K 31/496

(54) **Nanoparticulate aripiprazole formulations**

(30) Priority: 15.09.2005 US 717325 P
(62) Divisional of application: 06814574.7
(71) Applicant: Elan Pharma International Limited, Athlone County Westmeath (IE)
(72) Inventor: The designation of the inventor has not yet been filed
(74) Representative: Wichmann, Hendrik

(57) **Abstract**

The present invention is directed to compositions and methods comprising nanoparticulate aripiprazole, or salts or derivatives thereof, having improved bioavailability, faster rates of absorption and a faster onset of therapeutic effect. The nanoparticulate aripiprazole compositions are proposed to have an average effective particle size of less than about 2000 nm, and may be useful for the treatment of diseases and disorders of the central nervous system, including mental diseases and disorders.

## Description

### CROSS-REFERENCE TO RELATED PATENT APPLICATIONS

This application claims the benefit under 35 U.S.C. § 119(e) to U.S. Provisional Application No. 60/717,325, filed on September 15,2005, which is incorporated by reference herein in its entirety.

### FIELD OF THE INVENTION

The present invention relates generally to compounds and compositions useful in the treatment of diseases and disorders of the central nervous system, such as mental diseases and disorders. More specifically, the invention relates to compositions comprising a nanoparticulate aripiprazole, or a salt or derivative thereof, having an effective average particle size of less than about 2000 nm. The invention also relates to nanoparticulate aripiprazole formulations, methods of manufacturing nanoparticulate aripiprazole compositions and methods of treatment using such compositions.

### BACKGROUND OF THE INVENTION

The following discussion of the background of the invention is merely provided to aid the reader in understanding the invention and is not admitted to describe or constitute prior art to the invention.

### A. Background Regarding Aripipriazole

Currently there are many drugs available for the treatment of disorders of the central nervous system ("CNS"), including drugs to treat mental diseases and disorders involving the CNS. Among these drugs is a type known as antipsychotics. Antipsychotics are often used for treating serious mental conditions such as schizophrenia, bipolar disorder, and schizophreniform illness.

Antipsychotics can be classified into three broad categories based on the underlying mechanism of action: typical anti-psychotics, atypical anti-psychotics and a newer category of drugs termed dopamine partial agonists.

Many antipsychotic drugs work, in general, by blocking the dopamine D2 receptors in the brain. These receptors are an important link in the dopamine pathway and are responsible for increasing or decreasing dopamine levels in the brain. Dopamine, a catecholamine neurotransmitter, has been shown to be essential for the normal functioning of the central nervous system; for example, reduced concentration of dopamine within the brain have been associated with Parkinson's disease, while an excess of dopamine may cause such conditions as schizophrenia.

Typical antipsychotics such as the phenothiazines (*e.g*., chlorpromazine, fluphenazine, perphenazine and prochlorperazine) block the D2 receptor, but are relatively non-specific and block receptors in other biochemical pathways as well (*e.g*., the nigrostrial, tuberoinfundibular and mesocortical pathways). Atypical antipsychotics (*e.g*., clozapine, olanzapine, quetiapine and ziprasidone) appear to be slightly more discriminating than the typical antipsychotics, and in addition to the D2 receptors, the atypicals have been shown to block serotonin receptors, such as the 5HT_{2A,C} and the 5HT_{1A} receptors.

The dopamine partial agonist antipsychotics such as aripiprazole are sometimes referred to as atypical antipsychotics. They are quite similar to the atypical antipsychotics in that they also act on both dopamine and serotonin receptors. Aripiprazole appears to mediate its antipsychotic effects primarily by partial agonism at the dopamine D2 receptor. This partial agonism at D2 receptors has been shown to modulate dopaminergic activity in areas where dopamine activity may be high or low, such as the mesolimbic and mesocortical areas of the schizophrenic brain, respectively. In addition to partial agonist activity at the D2 receptor, aripiprazole is also a partial agonist at the 5-HT_{1A} receptor, and like the other atypical antipsychotics, aripiprazole displays an antagonist profile at the 5-HT_{2A} receptor as well. Aripiprazole also has moderate affinity for histamine and alpha-adrenergic receptors, but no appreciable affinity for cholinergic muscarinic receptors.

Aripiprazole, also known as a psychotropic drug, is indicated for the treatment of schizophrenia and acute manic and mixed episodes associated with bipolar disorder.

Aripiprazole, chemically known as 7-[4-[4-(2,3-dichlorophenyl)-1-piperazinyl]butoxy]-3,4-dihydrocarbostyril, has the empirical formula of C₂₃H₂₇Cl₂N₃O₂ and molecular weight of 448.38. The chemical structure of aripiprazole is shown below:

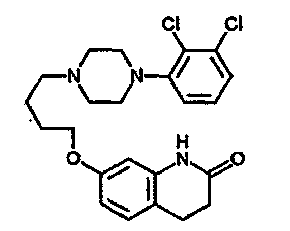

Aripiprazole is commercially available in the United States under the brand name Abilify®, manufactured/marketed by Bristol-Myers Squibb of Princeton, New Jersey and marketed by Otsuka America Pharmaceutical, Inc. It is available in tablet form for oral administration in dosage strengths of 5 mg, 10 mg, 15mg, and 30 mg per tablet. Inactive ingredients of the tablets include lactose monohydrate, cornstarch, microcrystalline cellulose, hydroxypropyl cellulose, and magnesium stearate. Colorants include ferric oxide (yellow or red) and FD&C Blue No. 2 Aluminum Lake.

Generally, aripiprazole is initially administered in amounts of 10 mg or 15 mg daily on a once-a-day schedule without regard to meals. Aripiprazole has been systematically evaluated and shown to be effective in a dose range of 10 mg/day to 30 mg/day. Increases in the dosing regimen occur after at least two weeks, the time needed to achieve a steady state plasma level.

Aripiprazole displays linear kinetics with an elimination half-life of approximately 75 hours, and steady state plasma concentrations are achieved in about 14 days. Cₘₐₓ is achieved in 3-5 hours after oral dosing, and the bioavailabilty of the oral tablets appears to be about 90%.

Aripiprazole and formulations thereof, have been described in, for example, U.S. Patent No. 4,734,416 to Banno et al. for "Pharmaceutically Useful Carbostyril Derivatives," U.S. Patent No. 5,006,528 to Oshiro et al. for "Carbostyril Derivatives," and U.S. Patent No. 6,884,768 to Kimura et al. for "Medicinal Compositions," U.S. Patent No. 6,977,257 to Parab et al. for "Aripiprazole Oral Solution," and U.S. Patent No. 6,995,264 to Tsujmori et al. for "Process for Preparing Aripiprazole."

Conventional, currently available antipsychotic drugs, including conventional formulations of aripiprazole, are often associated with undesirable side effects, some of which can be severe and debilitating. For example, many patients suffer from drug-induced extrapyramidal symptoms which include drug-induced Parkinsonism, acute dystonic reactions, akathisia, tardive dyskinesia and tardive dystonia (*e.g*., as determined by The Simpson Angus Scale, and/or the Barnes Akathisia Rating Scale and Abnormal Involuntary Movement Scale (AIMS), well known scales for assessing extra pyramidal symptoms). Unfortunately, the great majority of drugs available for treatment of CNS disorders (*e.g*., schizophrenia and bipolar disorder) are prone to produce these extra pyramidal side effects when used at dosages that yield a beneficial effect on the symptoms of the disease. Additionally, many drugs are associated with a sedative effect or may have an undesirable influence on the affective symptoms of the disease, causing, for example, depression. And in some instance, long term use of the drug leads to irreversible conditions, such as the tardive dyskinesia and tardive dystonia referred to above.

Furthermore, many patients do not respond or only partially respond to the present drug treatments, and estimates of such partial- or non-responders vary between 40% and 80% of those treated. The severity of adverse events, the lack of efficacy in a considerable number of patients, and the fact that many of the patients in need of these drugs are not in full control of their mental faculties, often results in poor patient compliance and thus diminished therapeutic effect.

Accordingly, there is a need for antipsychotic drug formulations that control or eliminate psychotic symptoms with fewer or diminished side effects, and which can be formulated to increase patient compliance.

### B. Background Regarding Nanoparticulate Compositions

Nanoparticulate compositions, first described in U.S. Patent No. 5,145,684 ("the '684 patent"), comprise particles of a poorly soluble therapeutic or diagnostic agent having a non-crosslinked surface stabilizer adsorbed onto or associated with the surface of the drug. The '684 patent also describes method of making such nanoparticulate active agent compositions but does not describe compositions comprising aripiprazole in nanoparticulate form. Methods of making nanoparticulate active agent compositions are described in, for example, U.S. Patent Nos. 5,518,187 and 5,862,999, both for "Method of Grinding Pharmaceutical Substances"; U.S. Patent No. 5,718,388, for "Continuous Method of Grinding Pharmaceutical Substances"; and U.S. Patent No. 5,510,118 for "Process of Preparing Therapeutic Compositions Containing Nanoparticles."

Nanoparticulate active agent compositions are also described, for example, in U.S. Patent Nos. 5,298,262 for "Use of Ionic Cloud Point Modifiers to Prevent Particle Aggregation During Sterilization"; 5,302,401 for "Method to Reduce Particle Size Growth During Lyophilization"; 5,318,767 for "X-Ray Contrast Compositions Useful in Medical Imaging"; 5,326,552 for "Novel Formulation For Nanoparticulate X-Ray Blood Pool Contrast Agents Using High Molecular Weight Non-ionic Surfactants"; 5,328,404 for "Method of X-Ray Imaging Using Iodinated Aromatic Propanedioates"; 5,336,507 for "Use of Charged Phospholipids to Reduce Nanoparticle Aggregation"; 5,340,564 for "Formulations Comprising Olin 10-G to Prevent Particle Aggregation and Increase Stability"; 5,346,702 for "Use of Non-Ionic Cloud Point Modifiers to Minimize Nanoparticulate Aggregation During Sterilization"; 5,349,957 for "Preparation and Magnetic Properties of Very Small Magnetic-Dextran Particles"; 5,352,459 for "Use of Purified Surface Modifiers to Prevent Particle Aggregation During Sterilization"; 5,399,363 and 5,494,683, both for "Surface Modified Anticancer Nanoparticles"; 5,401,492 for "Water Insoluble NonMagnetic Manganese Particles as Magnetic Resonance Enhancement Agents"; 5,429,824 for "Use of Tyloxapol as a Nanoparticulate Stabilizer"; 5,447,710 for "Method for Making Nanoparticulate X-Ray Blood Pool Contrast Agents Using High Molecular Weight Non-ionic Surfactants"; 5,451,393 for "X-Ray Contrast Compositions Useful in Medical Imaging"; 5,466,440 for "Formulations of Oral Gastrointestinal Diagnostic X-Ray Contrast Agents in Combination with Pharmaceutically Acceptable Clays"; 5,470,583 for "Method of Preparing Nanoparticle Compositions Containing Charged Phospholipids to Reduce Aggregation"; 5,472,683 for "Nanoparticulate Diagnostic Mixed Carbamic Anhydrides as X-Ray Contrast Agents for Blood Pool and Lymphatic System Imaging"; 5,500,204 for "Nanoparticulate Diagnostic Dimers as X-Ray Contrast Agents for Blood Pool and Lymphatic System Imaging"; 5,518,738 for "Nanoparticulate NSAID Formulations"; 5,521,218 for "Nanoparticulate Iododipamide Derivatives for Use as X-Ray Contrast Agents"; 5,525,328 for "Nanoparticulate Diagnostic Diatrizoxy Ester X-Ray Contrast Agents for Blood Pool and Lymphatic System Imaging"; 5,543,133 for "Process of Preparing X-Ray Contrast Compositions Containing Nanoparticles"; 5,552,160 for "Surface Modified NSAID Nanoparticles"; 5,560,931 for "Formulations of Compounds as Nanoparticulate Dispersions in Digestible Oils or Fatty Acids"; 5,565,188 for "Polyalkylene Block Copolymers as Surface Modifiers for Nanoparticles"; 5,569,448 for "Sulfated Non-ionic Block Copolymer Surfactant as Stabilizer Coatings for Nanoparticle Compositions"; 5,571,536 for "Formulations of Compounds as Nanoparticulate Dispersions in Digestible Oils or Fatty Acids"; 5,573,749 for "Nanoparticulate Diagnostic Mixed Carboxylic Anydrides as X-Ray Contrast Agents for Blood Pool and Lymphatic System Imaging"; 5,573,750 for "Diagnostic Imaging X-Ray Contrast Agents"; 5,573,783 for "Redispersible Nanoparticulate Film Matrices With Protective Overcoats"; 5,580,579 for "Site-specific Adhesion Within the GI Tract Using Nanoparticles Stabilized by High Molecular Weight, Linear Poly(ethylene Oxide) Polymers"; 5,585,108 for "Formulations of Oral Gastrointestinal Therapeutic Agents in Combination with Pharmaceutically Acceptable Clays"; 5,587,143 for "Butylene Oxide-Ethylene Oxide Block Copolymers Surfactants as Stabilizer Coatings for Nanoparticulate Compositions"; 5,591,456 for "Milled Naproxen with Hydroxypropyl Cellulose as Dispersion Stabilizer"; 5,593,657 for "Novel Barium Salt Formulations Stabilized by Non-ionic and Anionic Stabilizers"; 5,622,938 for "Sugar Based Surfactant for Nanocrystals"; 5,628,981 for "Improved Formulations of Oral Gastrointestinal Diagnostic X-Ray Contrast Agents and Oral Gastrointestinal Therapeutic Agents"; 5,643,552 for "Nanoparticulate Diagnostic Mixed Carbonic Anhydrides as X-Ray Contrast Agents for Blood Pool and Lymphatic System Imaging"; 5,718,388 for "Continuous Method of Grinding Pharmaceutical Substances"; 5,718,919 for "Nanoparticles Containing the R(-)Enantiomer of Ibuprofen"; 5,747,001 for "Aerosols Containing Beclomethasone Nanoparticle Dispersions"; 5,834,025 for "Reduction of Intravenously Administered Nanoparticulate Formulation Induced Adverse Physiological Reactions"; 6,045,829 "Nanocrystalline Formulations of Human Immunodeficiency Virus (HIV) Protease Inhibitors Using Cellulosic Surface Stabilizers"; 6,068,858 for "Methods of Making Nanocrystalline Formulations of Human Immunodeficiency Virus (HTV) Protease Inhibitors Using Cellulosic Surface Stabilizers"; 6,153,225 for "Injectable Formulations of Nanoparticulate Naproxen"; 6,165,506 for "New Solid Dose Form of Nanoparticulate Naproxen"; 6,221,400 for "Methods of Treating Mammals Using Nanocrystalline Formulations of Human Immunodeficiency Virus (HIV) Protease Inhibitors"; 6,264,922 for "Nebulized Aerosols Containing Nanoparticle Dispersions"; 6,267,989 for "Methods for Preventing Crystal Growth and Particle Aggregation in Nanoparticle Compositions"; 6,270,806 for "Use of PEG-Derivatized Lipids as Surface Stabilizers for Nanoparticulate Compositions"; 6,316,029 for "Rapidly Disintegrating Solid Oral Dosage Form," 6,375,986 for "Solid Dose Nanoparticulate Compositions Comprising a Synergistic Combination of a Polymeric Surface Stabilizer and Dioctyl Sodium Sulfosuccinate"; 6,428,814 for "Bioadhesive Nanoparticulate Compositions Having Cationic Surface Stabilizers"; 6,431,478 for "Small Scale Mill"; 6,432,381 for "Methods for Targeting Drug Delivery to the Upper and/or Lower Gastrointestinal Tract," U.S. Patent No. 6,582,285 for "Apparatus for Sanitary Wet Milling"; and U.S. Patent No. 6,592,903 for "Nanoparticulate Dispersions Comprising a Synergistic Combination of a Polymeric Surface Stabilizer and Dioctyl Sodium Sulfosuccinate"; 6,656,504 for "Nanoparticulate Compositions Comprising Amorphous Cyclosporine"; 6,742,734 for "System and Method for Milling Materials"; 6,745,962 for "Small Scale Mill and Method Thereof'; 6,811,767 for "Liquid Droplet Aerosols of Nanoparticulate Drugs"; 6,908,626 for "Compositions Having a Combination of Immediate Release and Controlled Release Characteristics"; 6,969,529 for "Nanoparticulate Compositions Comprising Copolymers of Vinyl Pyrrolidone and Vinyl Acetate as Surface Stabilizers"; 6,976,647 for "System and Method for Milling Materials"; and 6,991,191 for "Method of Using a Small Scale Mill"; all of which are specifically incorporated by reference.

In addition, U.S. Patent Publication No. 20020012675 A1, for "Controlled Release Nanoparticulate Compositions"; U.S. Patent Publication No. 20050276974 for "Nanoparticulate Fibrate Formulations"; U.S. Patent Publication No. 20050238725 for "Nanoparticulate Compositions Having a Peptide as a Surface Stabilizer"; U.S. Patent Publication No. 20050233001 for "Nanoparticulate Megestrol Formulations"; U.S. Patent Publication No. 20050147664 for "Compositions Comprising Antibodies and Methods of Using the Same for Targeting Nanoparticulate Active Agent Delivery"; U.S. Patent Publication No. 20050063913 for "Novel Metaxalone Compositions"; U.S. Patent Publication No. 20050042177 for "Novel Compositions of Sildenafil Free Base"; U.S. Patent Publication No. 20050031691 for "Gel Stabilized Nanoparticulate Active Agent Compositions"; U.S. Patent Publication No. 20050019412 for " Novel Glipizide Compositions"; U.S. Patent Publication No. 20050004049 for "Novel Griseofulvin Compositions"; U.S. Patent Publication No. 20040258758 for "Nanoparticulate Topiramate Formulations"; U.S. Patent Publication No. 20040258757 for " Liquid Dosage Compositions of
Stable Nanoparticulate Active Agents"; U.S. Patent Publication No. 20040229038 for "Nanoparticulate Meloxicam Formulations"; U.S. Patent Publication No. 20040208833 for "Novel Fluticasone Formulations"; U.S. Patent Publication No. 20040195413 for " Compositions and Method for Milling Materials"; U.S. Patent Publication No. 20040156895 for "Solid Dosage Forms Comprising Pullulan"; U.S. Patent Publication No. 20040156872 for "Novel Nimesulide Compositions"; U.S. Patent Publication No. 20040141925 for "Novel Triamcinolone Compositions"; U.S. Patent Publication No. 20040115134 for "Novel Nifedipine Compositions"; U.S. Patent Publication No. 20040105889 for "Low Viscosity Liquid Dosage Forms"; U.S. Patent Publication No. 20040105778 for "Gamma Irradiation of Solid Nanoparticulate Active Agents"; U.S. Patent Publication No. 20040101566 for "Novel Benzoyl Peroxide Compositions"; U.S. Patent Publication No. 20040057905 for "Nanoparticulate Beclomethasone Dipropionate Compositions"; U.S. Patent Publication No. 20040033267 for "Nanoparticulate Compositions of Angiogenesis Inhibitors"; U.S. Patent Publication No. 20040033202 for "Nanoparticulate Sterol Formulations and Novel Sterol Combinations"; U.S. Patent Publication No. 20040018242 for "Nanoparticulate Nystatin Formulations"; U.S. Patent Publication No. 20040015134 for "Drug Delivery Systems and Methods"; U.S. Patent Publication No. 20030232796 for "Nanoparticulate Polycosanol Formulations & Novel Polycosanol Combinations"; U.S. Patent Publication No. 20030215502 for "Fast Dissolving Dosage Forms Having Reduced Friability"; U.S. Patent Publication No. 20030185869 for "NanoparEiculate Compositions Having Lysozyme as a Surface Stabilizer"; U.S. Patent Publication No. 20030181411 for "Nanoparticulate Compositions of Mitogen-Activated Protein (MAP) Kinase Inhibitors"; U.S. Patent Publication No. 20030137067 for "Compositions Having a Combination of Immediate Release and Controlled Release Characteristics"; U.S. Patent Publication No. 20030108616 for "Nanoparticulate Compositions Comprising Copolymers of Vinyl Pyrrolidone and Vinyl Acetate as Surface Stabilizers"; U.S. Patent Publication No. 20030095928 for "Nanoparticulate Insulin"; U.S. Patent Publication No. 20030087308 for "Method for High Through- put Screening Using a Small Scale Mill or Microfluidics"; U.S. Patent Publication No. 20030023203 for "Drug Delivery Systems & Methods"; U.S. Patent Publication No. 20020179758 for "System and Method for Milling Materials"; and U.S. Patent Publication No. 20010053664 for "Apparatus for Sanitary Wet Milling," describe nanoparticulate active agent compositions and are specifically incorporated by reference. None of these references describe compositions of nanoparticulate aripiprazole.

Amorphous small particle compositions are described, for example, in U.S. Patent Nos. 4,783,484 for "Particulate Composition and Use Thereof as Antimicrobial Agent"; U.S. Patent No. 4,826,689 for "Method for Making Uniformly Sized Particles from Water-Insoluble Organic Compounds"; U.S. Patent No. 4,997,454 for "Method for Making Uniformly-Sized Particles From Insoluble Compounds"; U.S. Patent No. 5,741,522 for "Ultrasmall, Non-aggregated Porous Particles of Uniform Size for Entrapping Gas Bubbles Within and Methods"; and U.S. Patent No. 5,776,496, for "Ultrasmall Porous Particles for Enhancing Ultrasound Back Scatter," all of which are specifically incorporated herein by reference.

Aripiprazole has high therapeutic value in the treatment of disorders of the CNS, such as mental diseases and disorders. However, due to the severity of adverse side-effects and associated patient compliance issues, the therapeutic outcome for treatments requiring aripiprazole may be compromised. Accordingly, there is a need in the art for aripiprazole compositions which overcome these and other problems associated with its use in the treatment of mental diseases and disorders. Compositions and methods directed to formulations of aripiprazole which exhibit enhanced bioavailability, increased dissolution rate, reduced drug dosage, reduced adverse side effects, and which may be administered to reduce or eliminate patient compliance problems would satisfy these needs.

### SUMMARY OF THE INVENTION

The compositions and methods described herein relate to compositions comprising aripiprazole, or a salt or derivative thereof, having an effective average particle size of less than about 2000 nm. In general, the compositions comprise particles of a nanoparticulate aripiprazole, and at least one surface stabilizer adsorbed or associated with the surface of the aripiprazole particles. Such nanoparticles may be in crystalline phase, an amorphous phase, a semi-crystalline phase, a semi-amorphous phase, or mixtures thereof.

Additionally, the compositions may comprise one or more surface stabilizers. For example, the compositions may comprise at least one primary and at least one secondary surface stabilizer. Exemplary surface stabilizers may include one or more of an anionic surface stabilizer, a cationic surface stabilizers, a non-ionic surface stabilizer, a zwitterionic surface stabilizer, and an ionic surface stabilizer.

In some embodiments, the compositions may additionally include one or more pharmaceutically acceptable excipients, carriers, active agents or combinations thereof. In some embodiments, active agents may include agents useful for the treatment of schizophrenia, bipolar disorder, schizophreniform illness and related conditions. By way of example but not by way of limitation, active agents may include phenothiazines, such as chlorpromazine, fluphenazine, perphanazine, prochlorperazine, thioridazine, trifluoperazine; butyrophenones such as olanzapine, risperidone, quetiapine, and ziprasidone and combinations thereof.

The nanoparticulate aripiprazole compositions described herein may be formulated for dosage or administration in a variety of forms, although in some embodiments, an injectable form may be preferred. For example, aripiprazole formulations suitable for intramuscular (IM) or subcutaneous (SC) administration may be preferred. In some embodiments, the injectable compositions may be formulated so as to form a depot of the aripiprazole upon injection. In this form, the aripiprazole may be slowly released with approximately zero order kinetics (*e.g*., at a constant rate) from the depot site for a given period of time, including but not limited to, greater than one week, such as from two weeks to twenty-four weeks, two weeks to twelve weeks, two weeks to six weeks.

Though any pharmaceutically acceptable dosage form may be utilized, dosage forms contemplated include but are not limited to formulations for oral, pulmonary, rectal, colonic, parenteral, intracisternal, intravaginal, intraperitoneal, ocular, otic, local, buccal, nasal, and topical administration. Dosage forms may include bioadhesives, liquid dispersions, gels, aerosols, ointments, creams, lyophilized formulations, tablets, and capsules, and dosage forms may also include controlled release formulations, fast melt formulations, delayed release formulations, extended release formulations, pulsatile release formulations, and mixed immediate release and controlled release formulations. Combinations of these dosage forms are also contemplated.

The nanoparticulate aripiprazole compositions disclosed herein are also contemplated to exhibit improved pharmacokinetic properties as compared to a non-nanoparticulate composition of the same aripiprazole.

In further embodiments, the pharmacokinetic profiles of the nanoparticulate aripiprazole compositions may be substantially similar when administered to a fed or fasted subject; in other embodiments, the nanoparticulate aripiprazole compositions may be bioequivalent when administered to a fed or fasted subject.

Additionally disclosed are methods related to making nanoparticulate aripiprazole compositions having an effective average particle size of less than about 2000 nm. By way of example, but not by way of limitation, methods may include contacting particles of the aripiprazole with at least one surface stabilizer for a time and under conditions sufficient to provide a nanoparticulate aripiprazole composition having an effective average particle size of less than about 2000 nm. In some methods, contacting may include, for example, milling, homogenization, freezing, template emulsion, precipitation, supercritical fluid techniques, or combinations thereof.

Also disclosed are methods of using the nanoparticulate aripiprazole formulations, for example, to treat or prevent diseases, disorders, symptoms or conditions in a subject. By way of example, but not by way of limitation, the compositions may be used to treat diseases or disorders of the central nervous system such as mental diseases and disorders. Exemplary mental diseases and disorders may include but are not limited to schizophrenia, bipolar disorder, schizophreniform illness and related conditions. In some embodiments, related conditions may include drug-induced extrapyramidal symptoms such as, but not limited to drug-induced Parkinsonism, acute dystonic reactions, akathisia, tardive dyskinesia and tardive dystonia.

Exemplary methods of treatment may include administering to a subject a stable nanoparticulate aripiprazole composition including aripiprazole or a derivative or a salt thereof and at least one surface stabilizer having an effective average particle size of less than about 200 nm. In some embodiments, the subject may have been diagnosed with a central nervous system disorder, such as a mental disease or disorder. In other embodiments, the compositions may be used to treat symptoms indicative of a CNS disease or disorder, such as a mental disease or disorder.

Both the foregoing summary of the invention and the following detailed description of the invention are exemplary and explanatory and are intended to provide further details of the invention as claimed. Other objects, advantages, and novel features will be readily apparent to those skilled in the art from the following detailed description of the invention.

### DETAILED DESCRIPTION OF INVENTION

### A. Nanoparticulate Aripiprazole Compositions

The nanoparticulate compositions described herein include an antipsychotic drug, such as aripiprazole or a salt or derivative thereof and at least one surface stabilizer associated with or adsorbed onto the surface of the drug. In some embodiments, the average effective particle size may be less than about 2000 nm.

As taught by the '684 patent, and as described in more detail below, not every combination of surface stabilizer and active agent will results in a stable nanoparticulate composition. It was surprisingly discovered that stable, nanoparticulate aripiprazole formulations can be made.

Advantages of the nanoparticulate aripiprazole compositions described herein, as compared to non-nanoparticulate aripiprazole compositions (*e.g*., microcrystalline or solubilized dosage forms) may include, but are not limited to: (1) smaller tablet or other solid dosage form size; (2) smaller doses of the drug required to obtain the same pharmacological effect, thus causing fewer or less sever side effects; (3) improved pharmacokinetic profiles; (4) increased bioavailability; (5) substantially similar pharmacokinetic profiles of the nanoparticulate aripiprazole compositions when administered in the fed versus the fasted state; (6) bioequivalency of the nanoparticulate aripiprazole compositions when administered in the fed versus the fasted state; (7) an increased rate of dissolution for the nanoparticulate aripiprazole compositions; and (8) the use of nanoparticulate aripiprazole compositions in conjunction with other active agents for the treatment of CNS diseases, disorders, symptoms, or conditions or to treat the side effects of antipsychotic drug therapy.

The compositions described herein may be formulated for administration for any pharmaceutically acceptable dosing form. In some embodiments, however, an injectable dosage form may be preferred (such as for intramuscular or subcutaneous injection), for example as a depot, to allow continued gradual release of the drug. Other dosage forms contemplated include but are not limited to parental injection (*e.g*., intravenous, intramuscular, or subcutaneous), oral administration in solid, liquid, bioadhesive or aerosol form, vaginal, nasal, rectal, ocular, local (powders, ointments, or drops), buccal, intracisternal, intraperitoneal, or topical administrations, and the like.

In other embodiments, the preferred dosage form may be a solid dosage form such as a tablet. Exemplary solid dosage forms include, but are not limited to, tablets, capsules, sachets, lozenges, powders, pills, or granules, and the solid dosage form can be, for example, a fast melt dosage form, controlled release dosage form, lyophilized dosage form, delayed release dosage form, extended release dosage form, pulsatile release dosage form, mixed immediate release and controlled release dosage form, or a combination thereof.

The methods and compositions described herein also relate to nanoparticulate aripiprazole compositions together with one or more non-toxic physiologically acceptable carriers, adjuvants, or vehicles, collectively referred to as carriers.

The present invention is described herein using several definitions, as set forth below and throughout the application.

As used herein, the term "subject" is used to mean an animal, preferably a mammal, including a human or non-human. The terms "patient" and subject may be used interchangeably.

The term "effective average particle size of less than about 2000 nm," as used herein, means that at least about 50% of the nanoparticulate aripiprazole particles have a size of less than about 2000 nm (by weight or by other suitable measurement technique, such as by number or by volume) when measured by, for example, sedimentation flow fractionation, photon correlation spectroscopy, light scattering, disk centrifugation, and other techniques known to those of skill in the art.

As used herein, "about" will be understood by persons of ordinary skill in the art and will vary to some extent on the context in which it is used. If there are uses of the term which are not clear to persons of ordinary skill in the art given the context in which it is used, "about" will mean up to plus or minus 10% of the particular term.

As used herein with reference to stable nanoparticulate aripiprazole, "stable" connotes, but is not limited to one or more of the following parameters: (1) the particles do not appreciably flocculate or agglomerate due to interparticle attractive forces or otherwise significantly increase in particle size over time; (2) that the physical structure of the particles is not altered over time, such as by conversion from an amorphous phase to a crystalline phase; (3) that the particles are chemically stable; and/or (4) where the aripiprazole has not been subject to a heating step at or above the melting point of the aripiprazole in the preparation of the nanoparticles of the present invention.

The term "conventional" or "non-nanoparticulate" active agent shall mean an active agent which is solubilized or which has an effective average particle size of greater than about 2000 nm. Nanoparticulate active agents as defined herein generally have an effective average particle size of less than about 2000 nm.

The phrase "poorly water soluble drugs" as used herein refers to those drugs that have a solubility in water of less than about 30 mg/ml, less than about 20 mg/ml, less than about 10 mg/ml, or less than about 1 mg/ml.

As used herein, the phrase "therapeutically effective amount" shall mean that drug dosage that provides the specific pharmacological response for which the drug is administered in a significant number of subjects in need of such treatment. It is emphasized that a therapeutically effective amount of a drug that is administered to a particular subject in a particular instance will not always be effective in treating the conditions/diseases described herein, even though such dosage is deemed to be a therapeutically effective amount by those of skill in the art.

The term "particulate" as used herein refers to a state of matter which is characterized by the presence of discrete particles, pellets, beads or granules irrespective of their size, shape or morphology. The term "multiparticulate" as used herein means a plurality of discrete or aggregated particles, pellets, beads, granules or mixtures thereof irrespective of their size, shape or morphology.

### B. Preferred Characteristics of the Nanoparticulate Aripiprazole Compositions

### 1. Increased Bioavailability

The compositions of the invention comprising a nanoparticulate aripiprazole, or a salt or derivative thereof, are proposed to exhibit increased bioavailability, and require smaller doses as compared to prior or conventional aripiprazole formulations.

In some embodiments, the nanoparticulate aripiprazole compositions, upon administration to a mammal, produce therapeutic results at a dosage which is less than that of a non-nanoparticulate dosage form of the same aripiprazole. In addition, the need for a smaller dosage may decrease or eliminate the severity, intensity or duration of side effects associated with conventional antipsychotic drug compositions.

### 2. Improved Pharmacokinetic Profiles

The nanoparticulate aripiprazole compositions described herein may also exhibit a desirable pharmacokinetic profile when administered to mammalian subjects. The desirable pharmacokinetic profile of the aripiprazole compositions preferably includes, but is not limited to: (1) a Cₘₐₓ for aripiprazole or a derivative or salt thereof, when assayed in the plasma of a mammalian subject following administration, that is preferably greater than the Cₘₐₓ for a non-nanoparticulate formulation of the same aripiprazole, administered at the same dosage; and/or (2) an AUC for aripiprazole or a derivative or a salt thereof, when assayed in the plasma of a mammalian subject following administration, that is preferably greater than the AUC for a non-nanoparticulate formulation of the same aripiprazole, administered at the same dosage; and/or (3) a Tₘₐₓ for aripiprazole or a derivative or a salt thereof, when assayed in the plasma of a mammalian subject following administration, that is preferably less than the Tₘₐₓ for a non-nanoparticulate formulation of the same aripiprazole, administered at the same dosage. The desirable pharmacokinetic profile, as used herein, is the pharmacokinetic profile measured after the initial dose of aripiprazole or derivative or a salt thereof.

In one embodiment, a composition comprising at least one nanoparticulate aripiprazole or a derivative or salt thereof exhibits in comparative pharmacokinetic testing with a non-nanoparticulate formulation of the same aripiprazole (*e.g*., Abilify®), administered at the same dosage, a Tₘₐₓ not greater than about 90%, not greater than about 80%, not greater than about 70%, not greater than about 60%, not greater than about 50%, not greater than about 30%, not greater than about 25%, not greater than about 20%, not greater than about 15%, not greater than about 10%, or not greater than about 5% of the Tₘₐₓ exhibited by the non-nanoparticulate aripiprazole formulation.

In another embodiment, the composition comprising at least one nanoparticulate aripiprazole or a derivative or salt thereof, exhibits in comparative pharmacokinetic testing with a non-nanoparticulate formulation of the same aripiprazole (*e.g*., Abilify), administered at the same dosage, a Cₘₐₓ which is at least about 50%, at least about 100%, at least about 200%, at least about 300%, at least about 400%, at least about 500%, at least about 600%, at least about 700%, at least about 800%, at least about 900%, at least about 1000%, at least about 1100%, at least about 1200%, at least about 1300%, at least about 1400%, at least about 1500%, at least about 1600%, at least about 1700%, at least about 1800%, or at least about 1900% greater than the Cₘₐₓ exhibited by the non-nanoparticulate aripiprazole formulation.

In yet another embodiment, the composition comprising at least one nanoparticulate aripiprazole or a derivative or salt thereof, exhibits in comparative pharmacokinetic testing with a non-nanoparticulate formulation of the same aripiprazole (*e.g*., Abilify), administered at the same dosage, an AUC which is at least about 25%, at least about 50%, at least about 75%, at least about 100%, at least about 125%, at least about 150%, at least about 175%, at least about 200%, at least about 225%, at least about 250%, at least about 275%, at least about 300%, at least about 350%, at least about 400%, at least about 450%, at least about 500%, at least about 550%, at least about 600%, at least about 750%, at least about 700%, at least about 750%, at least about 800%, at least about 850%, at least about 900%, at least about 950%, at least about 1000%, at least about 1050%, at least about 1100%, at least about 1150%, or at least about 1200% greater than the AUC exhibited by the non-nanoparticulate aripiprazole formulation.

### 3. The Pharmacokinetic Profiles of the Aripiprazole Compositions are Not Affected by the Fed or Fasted State of the Subject Ingesting the Compositions

In some embodiments, the pharmacokinetic profile of the nanoparticulate aripiprazole compositions are not substantially affected by the fed or fasted state of a subject ingesting the composition. This means that there would be little or no appreciable difference in the quantity of drug absorbed or the rate of drug absorption when the nanoparticulate aripiprazole compositions are administered in the fed or fasted state.

Benefits of a dosage form which substantially eliminates the effect of food include an increase in subject convenience, thereby increasing subject compliance, as the subject does not need to ensure that they are taking a dose either with or without food. This is significant, as with poor subject compliance an increase in the medical condition for which the drug is being prescribed may be observed.

### 4. Bioequivalency of Aripiprazole Compositions When Administered in the Fed Versus the Fasted State

In some embodiments, administration of a nanoparticulate aripiprazole composition to a subject in a fasted state is bioequivalent to administration of the composition to a subject in a fed state. The difference in absorption of the nanoparticulate aripiprazole compositions, when administered in the fed versus the fasted state, preferably is less than about 100%, less than about 90%, less than about 80%, less than about 70%, less than about 60%, less than about 55%, less than about 50%, less than about 45%, less than about 40%, less than about 35%, less than about 30%, less than about 25%, less than about 20%, less than about 15%, less than about 10%, less than about 5%, or less than about 3%.

In some embodiments, the invention encompasses compositions comprising at least one nanoparticulate aripiprazole, wherein administration of the composition to a subject in a fasted state is bioequivalent to administration of the composition to a subject in a fed state, in particular as defined by Cₘₐₓ and AUC guidelines given by the U.S. Food and Drug Administration and the corresponding European regulatory agency (EMEA). Under U.S. FDA guidelines, two products or methods are bioequivalent if the 90% Confidence Intervals (CI) for AUC and Cₘₐₓ are between 0.80 to 1.25 (Tₘₐₓ measurements are not relevant to bioequivalence for regulatory purposes). To show bioequivalency between two compounds or administration conditions pursuant to Europe's EMEA guidelines, the 90% CI for AUC must be between 0.80 to 1.25 and the 90% CI for Cₘₐₓ must between 0.70 to 1.43.

### 5. Dissolution Profiles of the Aripiprazole Compositions

The nanoparticulate aripiprazole compositions are proposed to have unexpectedly dramatic dissolution profiles. Rapid dissolution of an administered active agent is preferable, as faster dissolution generally leads to faster onset of action and greater bioavailability. Additionally, a faster dissolution rate would allow for a larger dose of the drug to be absorbed, which would increase drug efficacy. To improve the dissolution profile and bioavailability of the aripiprazole, it would be useful to increase the drug's dissolution so that it could attain a level close to 100%.

The aripiprazole compositions of the invention preferably have a dissolution profile in which within about 5 minutes at least about 20% of the composition is dissolved. In other embodiments, at least about 30% or at least about 40% of the aripiprazole composition is dissolved within about 5 minutes. In yet other embodiments, preferably at least about 40%, at least about 50%, at least about 60%, at least about 70%, or at least about 80% of the aripiprazole composition is dissolved within about 10 minutes. In further embodiments, preferably at least about 70%, at least about 80%, at least about 90%, or at least about 100% of the aripiprazole composition is dissolved within 20 minutes.

In some embodiments, dissolution is preferably measured in a medium which is discriminating. Such a dissolution medium will produce two very different dissolution curves for two products having very different dissolution profiles in gastric juices; *i.e*., the dissolution medium is predictive of *in vivo* dissolution of a composition. An exemplary dissolution medium is an aqueous medium containing the surfactant sodium lauryl sulfate at 0.025 M. Determination of the amount dissolved can be carried out by spectrophotometry. The rotating blade method (European Pharmacopoeia) can be used to measure dissolution.

### 6. Redispersibility of the Aripiprazole Compositions of the Invention

An additional feature of the aripiprazole compositions described herein may include redispersion such that the effective average particle size of the redispersed aripiprazole particles is less than about 2 microns. This is significant, as if upon administration the aripiprazole compositions of the invention did not redisperse to a substantially nanoparticulate size, then the dosage form may lose the benefits afforded by formulating the aripiprazole into a nanoparticulate size.

Not wishing to be bound by any theory, it is proposed that nanoparticulate active agent compositions benefit from the small particle size of the active agent; if the active agent does not redisperse into the small particle sizes upon administration, then "clumps" or agglomerated active agent particles are formed, owing to the extremely high surface free energy of the nanoparticulate system and the thermodynamic driving force to achieve an overall reduction in free energy. With the formation of such agglomerated particles, the bioavailability of the dosage form may fall.

Moreover, the nanoparticulate aripiprazole compositions of the invention exhibit dramatic redispersion of the nanoparticulate aripiprazole particles upon administration to a mammal, such as a human or animal, as demonstrated by reconstitution/redispersion in a biorelevant aqueous media such that the effective average particle size of the redispersed aripiprazole particles is less than about 2 microns. Such biorelevant aqueous media can be any aqueous media that exhibit the desired ionic strength and pH, which form the basis for the biorelevance of the media. The desired pH and ionic strength are those that are representative of physiological conditions found in the human body. Such biorelevant aqueous media can be, for example, water, aqueous electrolyte solutions or aqueous solutions of any salt, acid, or base, or a combination thereof, which exhibit the desired pH and ionic strength. Such redispersion in a biorelevant media is predictive of in vivo efficacy of the aripiprazole dosage form.

Biorelevant pH is well known in the art. For example, in the stomach, the pH ranges from slightly less than 2 (but typically greater than 1) up to 4 or 5. In the small intestine the pH can range from 4 to 6, and in the colon it can range from 6 to 8. Biorelevant ionic strength is also well known in the art. Fasted state gastric fluid has an ionic strength of about 0.1M while fasted state intestinal fluid has an ionic strength of about 0.14. See *e.g*., Lindahl et al., "Characterization of Fluids from the Stomach and Proximal Jejunum in Men and Women," Pharm. Res., 14 (4): 497-502 (1997).

It is believed that the pH and ionic strength of the test solution is more critical than the specific chemical content. Accordingly, appropriate pH and ionic strength values can be obtained through numerous combinations of strong acids, strong bases, salts, single or multiple conjugate acid-base pairs (*i.e*., weak acids and corresponding salts of that acid), monoprotic and polyprotic electrolytes, etc.

Representative electrolyte solutions can be, but are not limited to, HCl solutions, ranging in concentration from about 0.001 to about 0.1 N, and NaCl solutions, ranging in concentration from about 0.001 to about 0.1 M, and mixtures thereof. For example, electrolyte solutions can be, but are not limited to, about 0.1 N HCl or less, about 0.01 N HCl or less, about 0.001 N HCl or less, about 0.1 M NaCl or less, about 0.01 M NaCl or less, about 0.001 M NaCl or less, and mixtures thereof. Of these electrolyte solutions, 0.01 M HCl and/or 0.1 M NaCl, are most representative of fasted human physiological conditions, owing to the pH and ionic strength conditions of the proximal gastrointestinal tract.

Electrolyte concentrations of 0.001 NHCl, 0.01 NHCl, and 0.1 NHCl correspond to pH 3, pH 2, and pH 1, respectively. Thus, a 0.01 N HCl solution simulates typical acidic conditions found in the stomach. A solution of 0.1 M NaCl provides a reasonable approximation of the ionic strength conditions found throughout the body, including the gastrointestinal fluids, although concentrations higher than 0.1 M may be employed to simulate fed conditions within the human GI tract.

Exemplary solutions of salts, acids, bases or combinations thereof, which exhibit the desired pH and ionic strength, include but are not limited to phosphoric acid/phosphate salts + sodium, potassium and calcium salts of chloride, acetic acid/acetate salts + sodium, potassium and calcium salts of chloride, carbonic acid/bicarbonate salts + sodium, potassium and calcium salts of chloride, and citric acid/citrate salts + sodium, potassium and calcium salts of chloride.

In other embodiments, the redispersed aripiprazole particles (*e.g*., redispersed in water, a biorelevant medium, or any other suitable dispersion medium) have an effective average particle size of less than about 2000 nm, less than about 1900 nm, less than about 1800 nm, less than about 1700 nm, less than about 1600 nm, less than about 1500 nm, less than about 1400 nm, less than about 1300 nm, less than about 1200 nm, less than about 1100 nm, less than about 1000 nm, less than about 900 nm, less than about 800 nm, less than about 700 nm, less than about 600 nm, less than about 500 nm, less than about 400 nm, less than about 300 nm, less than about 250 nm, less than about 200 nm, less than about 150 nm, less than about 100 nm, less than about 75 nm, or less than about 50 nm, as measured by light-scattering methods, microscopy, or other appropriate methods.

In still other embodiments, the redispersed aripiprazole particles, when administered to a mammal, redisperse such that the particles have an effective average particle size of less than about 2000 nm, less than about 1900 nm, less than about 1800 nm, less than about 1700 nm, less than about 1600 nm, less than about 1500 nm, less than about 1400 nm, less than about 1300 nm, less than about 1200 nm, less than about 1100 nm, less than about 1000 nm, less than about 900 nm, less than about 800 nm, less than about 700 nm, less than about 600 nm, less than about 500 nm, less than about 400 nm, less than about 300 nm, less than about 250 nm, less than about 200 nm, less than about 150 nm, less than about 100 nm, less than about 75 nm, or less than about 50 nm, as measured by light-scattering methods, microscopy, or other appropriate methods.

Redispersibility can be tested using any suitable means known in the art. *See e.g*., the example sections of U.S. Patent No. 6,375,986 for "Solid Dose Nanoparticulate Compositions Comprising a Synergistic Combination of a Polymeric Surface Stabilizer and Dioctyl Sodium Sulfosuccinate."

### 7. Aripiprazole Compositions Used in

### Conjunction with Other Active Agents

The compositions comprising a nanoparticulate aripiprazole, or a salt or derivative thereof, can additionally comprise one or more compounds useful in the treatment of diseases or disorders of the CNS, such as mental disease or disorders. Additionally, one or more compounds useful in the treatment of adverse antipsychotic drug side-effects are also contemplated. Examples of some compounds include, but are not limited to one or more of phenothiazines, such as chlorpromazine, fluphenazine, perphanazine, prochlorperazine, thioridazine, trifluoperazine; butyrophenones such as olanzapine, risperidone, quetiapine, and ziprasidone.

### C. Nanoparticulate Aripiprazole Compositions

The invention provides compositions comprising aripiprazole particles and at least one surface stabilizer. The surface stabilizers preferably are adsorbed on, or associated with, the surface of the aripiprazole particles. In some embodiments, surface stabilizers preferably physically adhere on, or associate with, the surface of the nanoparticulate aripiprazole particles, but do not chemically react with the aripiprazole particles or itself. In other embodiments, individually adsorbed molecules of the surface stabilizer are essentially free of intermolecular crosslinkages.

The present invention also includes aripiprazole compositions together with one or more non-toxic physiologically acceptable carriers, adjuvants, or vehicles, collectively referred to as carriers. The compositions can be formulated for parenteral injection (*e.g*., intravenous, intramuscular, or subcutaneous), oral administration in solid, liquid, or aerosol form, vaginal, nasal, rectal, ocular, local (powders, ointments or drops), buccal, intracisternal, intraperitoneal, topical or bioadhesive administration, and the like.

### 1. Aripiprazole Particles

The compositions of the invention comprise particles of aripiprazole or a salt or derivative thereof. The particles may be in crystalline phase, semi-crystalline phase, amorphous phase, semi-amorphous phase, or a combination thereof.

### 2. Surface Stabilizers

The choice of a surface stabilizer for aripiprazole is non-trivial and required extensive experimentation to realize a desirable formulation. Accordingly, the present invention is directed to the surprising discovery that stabilized nanoparticulate aripiprazole compositions can be made.

Combinations of more than one surface stabilizers may be used in the invention. Useful surface stabilizers which can be employed in the invention include, but are not limited to, known organic and inorganic pharmaceutical excipients. Such excipients include various polymers, low molecular weight oligomers, natural products, and surfactants. Surface stabilizers include nonionic, anionic, cationic, ionic, and zwitterionic surfactants.

Representative examples of surface stabilizers include hydroxypropyl methylcellulose (now known as hypromellose), hydroxypropylcellulose, polyvinylpyrrolidone, sodium lauryl sulfate, dioctylsulfosuccinate, gelatin, casein, lecithin (phosphatides), dextran, gum acacia, cholesterol, tragacanth, stearic acid, benzalkonium chloride, calcium stearate, glycerol monostearate, cetostearyl alcohol, cetomacrogol emulsifying wax, sorbitan esters, polyoxyethylene alkyl ethers (*e.g*., macrogol ethers such as cetomacrogol 1000), polyoxyethylene castor oil derivatives, polyoxyethylene sorbitan fatty acid esters (*e.g*., the commercially available Tweens^{®} such as *e.g*., Tween 20^{®} and Tween 80^{®} (ICI Speciality Chemicals)); polyethylene glycols (*e.g*., Carbowaxs 3550^{®} and 934^{®} (Union Carbide)), polyoxyethylene stearates, colloidal silicon dioxide, phosphates, carboxymethylcellulose calcium, carboxymethylcellulose sodium, methylcellulose, hydroxyethylcellulose, hypromellose phthalate, noncrystalline cellulose, magnesium aluminium silicate, triethanolamine, polyvinyl alcohol (PVA), 4-(1,1,3,3-tetramethylbutyl)-phenol polymer with ethylene oxide and formaldehyde (also known as tyloxapol, superione, and triton), poloxamers (*e.g*., Pluronics F68^{®} and F108^{®}, which are block copolymers of ethylene oxide and propylene oxide); poloxamines (*e.g*., Tetronic 908^{®}, also known as Poloxamine 908^{®}, which is a tetrafunctional block copolymer derived from sequential addition of propylene oxide and ethylene oxide to ethylenediamine (BASF Wyandotte Corporation, Parsippany, N.J.)); Tetronic 1508^{®} (T-1508) (BASF Wyandotte Corporation), Tritons X-200^{®}, which is an alkyl aryl polyether sulfonate (Rohm and Haas); Crodestas F-110^{®}, which is a mixture of sucrose stearate and sucrose distearate (Croda Inc.); p-isononylphenoxypoly-(glycidol), also known as Olin-lOG^{®} or Surfactant 10-G^{®} (Olin Chemicals, Stamford, CT); Crodestas SL-40^{®} (Croda, Inc.); and SA9OHCO, which is C₁₈H₃₇CH₂(CON(CH₃)-CH₂(CHOH)₄(CH₂0H)₂ (Eastman Kodak Co.); decanoyl-N-methylglucamide; n-decyl β-D-glucopyranoside; n-decyl β-D-maltopyranoside; n-dodecyl β-D-glucopyranoside; n-dodecyl β-D-maltoside; heptanoyl-N-methylglucamide; n-heptyl-β-D-glucopyranoside; n-heptyl β-D-thioglucoside; n-hexyl β-D-glucopyranoside; nonanoyl-N-methylglucamide; n-noyl β-D-glucopyranoside; octanoyl-N-methylglucamide; n-octyl-β-D-glucopyranoside; octyl β-D-thioglucopyranoside; PEG-phospholipid, PEG-cholesterol, PEG-cholesterol derivative, PEG-vitamin A, PEG-vitamin E, lysozyme, random copolymers of vinyl pyrrolidone and vinyl acetate, and the like.

Examples of useful cationic surface stabilizers include, but are not limited to, polymers, biopolymers, polysaccharides, cellulosics, alginates, phospholipids, and nonpolymeric compounds, such as zwitterionic stabilizers, poly-n-methylpyridinium, anthryul pyridinium chloride, cationic phospholipids, chitosan, polylysine, polyvinylimidazole, polybrene, polymethylmethacrylate trimethylammoniumbromide bromide (pMMTMABr), hexyldesyltrimethylammonium bromide (HDMAB), and polyvinylpyrrolidone-2-dimethylaminoethyl methacrylate dimethyl sulfate.

Other useful cationic stabilizers include, but are not limited to, cationic lipids, sulfonium, phosphonium, and quarternary ammonium compounds, such as stearyltrimethylammonium chloride, benzyl-di(2-chloroethyl)ethylammonium bromide, coconut trimethyl ammonium chloride or bromide, coconut methyl dihydroxyethyl ammonium chloride or bromide, decyl triethyl ammonium chloride, decyl dimethyl hydroxyethyl ammonium chloride or bromide, C₁₂₋₁₅dimethyl hydroxyethyl ammonium chloride or bromide, coconut dimethyl hydroxyethyl ammonium chloride or bromide, myristyl trimethyl ammonium methyl sulphate, lauryl dimethyl benzyl ammonium chloride or bromide, lauryl dimethyl (ethenoxy)₄ ammonium chloride or bromide, N-alkyl (C₁₂₋₁₈)dimethylbenzyl ammonium chloride, N-alkyl (C₁₄₋₁₈)dimethyl-benzyl ammonium chloride, N-tetradecylidmethylbenzyl ammonium chloride monohydrate, dimethyl didecyl ammonium chloride, N-alkyl and (C₁₂₋₁₄) dimethyl 1-napthylmethyl ammonium chloride, trimethylammonium halide, alkyl-trimethylammonium salts and dialkyl-dimethylammonium salts, lauryl trimethyl ammonium chloride, ethoxylated alkyamidoalkyldialkylammonium salt and/or an ethoxylated trialkyl ammonium salt, dialkylbenzene dialkylammonium chloride, N-didecyldimethyl ammonium chloride, N-tetradecyldimethylbenzyl ammonium, chloride monohydrate, N-alkyl(C₁₂₋₁₄) dimethyl 1-naphthylmethyl ammonium chloride and dodecyldimethylbenzyl ammonium chloride, dialkyl benzenealkyl ammonium chloride, lauryl trimethyl ammonium chloride, alkylbenzyl methyl ammonium chloride, alkyl benzyl dimethyl ammonium bromide, C₁₂, C₁₅, C₁₇ trimethyl ammonium bromides, dodecylbenzyl triethyl ammonium chloride, polydiallyldimethylammonium chloride (DADMAC), dimethyl ammonium chlorides, alkyldimethylammonium halogenides, tricetyl methyl ammonium chloride, decyltrimethylammonium bromide, dodecyltriethylammonium bromide, tetradecyltrimethylammonium bromide, methyl trioctylammonium chloride (ALIQUAT 336™), POLYQUAT 10™, tetrabutylammonium bromide, benzyl trimethylammonium bromide, choline esters (such as choline esters of fatty acids), benzalkonium chloride, stearalkonium chloride compounds (such as stearyltrimonium chloride and Di-stearyldimonium chloride), cetyl pyridinium bromide or chloride, halide salts of quaternized polyoxyethylalkylamines, MIRAPOL™ and ALKAQUAT™ (Alkaril Chemical Company), alkyl pyridinium salts; amines, such as alkylamines, dialkylamines, alkanolamines, polyethylenepolyamines, N,N-dialkylaminoalkyl acrylates, and vinyl pyridine, amine salts, such as lauryl amine acetate, stearyl amine acetate, alkylpyridinium salt, and alkylimidazolium salt, and amine oxides; imide azolinium salts; protonated quaternary acrylamides; methylated quaternary polymers, such as poly[diallyl dimethylammonium chloride] and poly-[N-methyl vinyl pyridinium chloride]; and cationic guar.

Such exemplary cationic surface stabilizers and other useful cationic surface stabilizers are described in J. Cross and E. Singer, Cationic Surfactants: Analytical and Biological Evaluation (Marcel Dekker, 1994); P. and D. Rubingh (Editor), Cationic Surfactants: Physical Chemistry (Marcel Dekker, 1991); and J. Richmond, Cationic Surfactants: Organic Chemistry, (Marcel Dekker, 1990).
Nonpolymeric surface stabilizers are any nonpolymeric compound, such benzalkonium chloride, a carbonium compound, a phosphonium compound, an oxonium compound, a halonium compound, a cationic organometallic compound, a quarternary phosphorous compound, a pyridinium compound, an anilinium compound, an ammonium compound, a hydroxylammonium compound, a primary ammonium compound, a secondary ammonium compound, a tertiary ammonium compound, and quarternary ammonium compounds of the formula NR₁R₂R₃R₄⁽⁺⁾. For compounds of the formula NR₁R₂R₃R₄⁽⁺⁾;

(i) none of R₁-R₄ are CH₃;

(ii) one of R₁-R₄ is CH₃;

(iii) three of R₁-R₄ are CH₃;

(iv) all of R₁-R₄ are CH₃;

(v) two of R₁-R₄ are CH₃, one of R₁-R₄ is C₆H₅CH₂, and one of R₁-R₄ is an alkyl chain of seven carbon atoms or less;

(vi) two of R₁-R₄ are CH₃, one of R₁-R₄ is C₆H₅CH₂, and one of R₁-R₄ is an alkyl chain of nineteen carbon atoms or more;

(vii) two of R₁-R₄ are CH₃ and one of R₁-R₄ is the group C₆H₅(CH₂)ₙ, where n>1;

(viii) two of R₁-R₄ are CH₃, one of R₁-R₄ is C₆H₅CH₂, and one of R₁-R₄ comprises at least one heteroatom;

(ix) two of R₁-R₄ are CH₃, one of R₁-R₄ is C₆H₅CH₂, and one of R₁₋R₄ comprises at least one halogen;

(x) two of R₁-R₄ are CH₃, one of R₁-R₄ is C₆H₅CH₂, and one of R₁-R₄ comprises at least one cyclic fragment;

(xi) two of R₁R₄ are CH₃ and one of R₁-R₄ is a phenyl ring; or

(xii) two of R₁-R₄ are CH₃ and two of R₁-R₄ are purely aliphatic fragments.

Such compounds include, but are not limited to, behenalkonium chloride, benzethonium chloride, cetylpyridinium chloride, behentrimonium chloride, lauralkonium chloride, cetalkonium chloride, cetrimonium bromide, cetrimonium chloride, cethylamine hydrofluoride, chlorallylmethenamine chloride (Quaternium-15), distearyldimonium chloride (Quaternium-5), dodecyl dimethyl ethylbenzyl ammonium chloride(Quaternium-14), Quaternium-22, Quaternium-26, Quaternium-18 hectorite, dimethylaminoethylchloride hydrochloride, cysteine hydrochloride, diethanolammonium POE (10) oletyl ether phosphate, diethanolammonium POE (3)oleyl ether phosphate, tallow alkonium chloride, dimethyl dioctadecylammoniumbentonite, stearalkonium chloride, domiphen bromide, denatonium benzoate, myristalkonium chloride, laurtrimonium chloride, ethylenediamine dihydrochloride, guanidine hydrochloride, pyridoxine HCl, iofetamine hydrochloride, meglumine hydrochloride, methylbenzethonium chloride, myrtrimonium bromide, oleyltrimonium chloride, polyquaternium-1, procainehydrochloride, cocobetaine, stearalkonium bentonite, stearalkoniumhectonite, stearyl trihydroxyethyl propylenediamine dihydrofluoride, tallowtrimonium chloride, and hexadecyltrimethyl ammonium bromide.

The surface stabilizers are commercially available and/or can be prepared by techniques known in the art. Most of these surface stabilizers are known pharmaceutical excipients and are described in detail in the Handbook of Pharmaceutical Excipients, published jointly by the American Pharmaceutical Association and The Pharmaceutical Society of Great Britain (The Pharmaceutical Press, 2000), specifically incorporated by reference.

In some embodiments, the surface stabilizers are copovidone (*e.g*., Plasdone® S630, which is random copolymer of vinyl acetate and vinyl pyrrolidone) and docusate sodium.

Povidone polymers are exemplary surface stabilizers useful in formulating an injectable nanoparticulate benidipine composition. Povidone polymers, also known as polyvidon(e), povidonum, PVP, and polyvinylpyrrolidone, are sold under the trade names Kollidon® (BASF Corp.) and Plasdone® (ISP Technologies, Inc.). They are polydisperse macromolecular molecules, with a chemical name of 1-ethenyl-2-pyrrolidinone polymers and 1-vinyl-2-pyrrolidinone polymers. Povidone polymers are produced commercially as a series of products having mean molecular weights ranging from about 10,000 to about 700,000 daltons. In some embodiments, preferred povidone polymers have a molecular weight of less than about 40,000 daltons; polymer size greater than 40,000 daltons might have difficulty clearing the body, and thus may be less useful as a surface modifier for a drug compound to be administered to a mammal.

Povidone polymers are prepared by, for example, Reppe's process, comprising: (1) obtaining 1,4-butanediol from acetylene and formaldehyde by the Reppe butadiene synthesis; (2) dehydrogenating the 1,4-butanediol over copper at 200° to form γ-butyrolactone; and (3) reacting γ-butyrolactone with ammonia to yield pyrrolidone. Subsequent treatment with acetylene gives the vinyl pyrrolidone monomer. Polymerization is carried out by heating in the presence of H₂O and NH₃. *See* The Merck Index, 10th Edition, pp. 7581 (Merck & Co., Rahway, NJ, 1983).

The manufacturing process for povidone polymers produces polymers containing molecules of unequal chain length, and thus different molecular weights. The molecular weights of the molecules vary about a mean or average for each particular commercially available grade. Because it is difficult to determine the polymer's molecular weight directly, the most widely used method of classifying various molecular weight grades is by K-values, based on viscosity measurements. The K-values of various grades of povidone polymers represent a function of the average molecular weight, and are derived from viscosity measurements and calculated according to Fikentscher's formula.

The weight-average of the molecular weight, Mw, is determined by methods that measure the weights of the individual molecules, such as by light scattering. Table 1 provides molecular weight data for several commercially available povidone polymers, all of which are soluble.

**TABLE 1**

| **Povidone** | **K-Value** | **Mv** **(Daltons)**** | **Mw** **(Daltons)**** | **Mn** **(Daltons)**** |
|---|---|---|---|---|
| Plasdone C-15^{®} | 17 ± 1 | 7,000 | 10,500 | 3,000 |
| Plasdone C-30^{®} | 30.5 ± 1.5 | 38,000 | 62,500* | 16,500 |
| Kollidon 12 | 11-14 | 3,900 | 2,000-3,000 | 1,300 |
| PF^{®} | | | | |
| Kollidon 17 | 16-18 | 9,300 | 7,000-11,000 | 2,500 |
| PF^{®} | | | | |
| Kollidon 25^{®} | 24-32 | 25,700 | 28,000-34,000 | 6,000 |

| | | | | |
|---|---|---|---|---|
| *Because the molecular weight is greater than 40,000 daltons, this povidone polymer may not be suitable for use as a surface stabilizer for a drug compound to be administered parenterally (*i.e*., injected). **Mv is the viscosity-average molecular weight, Mn is the number-average molecular weight, and Mw is the weight average molecular weight. Mw and Mn were determined by light scattering and ultra-centrifugation, and Mv was determined by viscosity measurements. | | | | |

Based on the data provided in Table 1, exemplary commercially available povidone polymers that may be useful in an Injectable composition include, but are not limited to, Plasdone C-15®, Kollidon 12 PF®, Kollidon 17 PF®, and Kollidon 25®.

### 3. Other Pharmaceutical Excipients

Pharmaceutical compositions according to the invention may also comprise one or more binding agents, filling agents, lubricating agents, suspending agents, sweeteners, flavoring agents, preservatives, buffers, wetting agents, disintegrants, effervescent agents, and other excipients. Such excipients are known in the art.

Examples of filling agents include lactose monohydrate, lactose anhydrous, and various starches; examples of binding agents are various celluloses and crosslinked polyvinylpyrrolidone, microcrystalline cellulose, such as Avicel^{®} PH101 and Avicel^{®} PH102, microcrystalline cellulose, and silicified microcrystalline cellulose (ProSolv SMCC™).

Suitable lubricants, including agents that act on the flowability of the powder to be compressed, include colloidal silicon dioxide, such as Aerosil^{®} 200, talc, stearic acid, magnesium stearate, calcium stearate, and silica gel.

Examples of sweeteners include any natural or artificial sweetener, such as sucrose, xylitol, sodium saccharin, cyclamate, aspartame, and acsulfame. Examples of flavoring agents include Magnasweet^{®} (trademark of MAFCO), bubble gum flavor, and fruit flavors, and the like.

Examples of preservatives include potassium sorbate, methylparaben, propylparaben, benzoic acid and its salts, other esters of parahydroxybenzoic acid such as butylparaben, alcohols such as ethyl or benzyl alcohol, phenolic compounds such as phenol, or quarternary compounds such as benzalkonium chloride.

Suitable diluents include pharmaceutically acceptable inert fillers, such as microcrystalline cellulose, lactose, dibasic calcium phosphate, saccharides, and/or mixtures of any of the foregoing. Examples of diluents include microcrystalline cellulose, such as Avicel^{®} PH101 and Avicel^{®} PH102; lactose such as lactose monohydrate, lactose anhydrous, and Pharmatose^{®} DCL21; dibasic calcium phosphate such as Emcompress^{®}; mannitol; starch; sorbitol; sucrose; and glucose.

Suitable disintegrants include lightly crosslinked polyvinyl pyrrolidone, corn starch, potato starch, maize starch, and modified starches, croscarmellose sodium, cross-povidone, sodium starch glycolate, and mixtures thereof.

Examples of buffers include phosphate buffer, citrate buffers and buffers made from other organic acids.

Examples of wetting or dispersing agents include a naturally-occurring phosphatide, for example, lecithin or condensation products of n-alkylene oxide with fatty acids, for example, polyoxyethylene stearate, or condensation products of ethylene oxide with long chain aliphatic alcohols, for example heptadecaethylene-oxycetanol, or condensation products of ethylene oxide with partial esters derived from fatty acids and a hexitol such as polyoxyethylene sorbitol mono-oleate, or condensation products of ethylene oxide with partial esters derived from fatty acids and hexitol anhydrides, for example, polyethylene sorbitan monooleate.

Examples of effervescent agents include effervescent couples such as an organic acid and a carbonate or bicarbonate. Suitable organic acids include, for example, citric, tartaric, malic, fumaric, adipic, succinic, and alginic acids and anhydrides and acid salts. Suitable carbonates and bicarbonates include, for example, sodium carbonate, sodium bicarbonate, potassium carbonate, potassium bicarbonate, magnesium carbonate, sodium glycine carbonate, L-lysine carbonate, and arginine carbonate. Alternatively, only the sodium bicarbonate component of the effervescent couple may be present.

### 4. Nanoparticulate Aripiprazole Particle Size

The compositions disclosed herein include nanoparticulate aripiprazole, wherein the aripiprazole particles may have an effective average particle size of less than about 2000 nm (*i.e*., 2 microns), less than about 1900 nm, less than about 1800 nm, less than about 1700 nm, less than about 1600 nm, less than about 1500 nm, less than about 1400 nm, less than about 1300 nm, less than about 1200 nm, less than about 1100 nm, less than about 1000 nm, less than about 900 nm, less than about 800 nm, less than about 700 nm, less than about 600 nm, less than about 500 nm, less than about 400 nm, less than about 300 nm, less than about 250 nm, less than about 200 nm, less than about 150 nm, less than about 100 nm, less than about 75 nm, or less than about 50 nm, as measured by light-scattering methods, microscopy, sedimentation field flow fractionation, photon correlation spectroscopy, disc centrifugation or other appropriate methods.

By "an effective average particle size of less than about 2000 nm" it is meant that at least 50% of the aripiprazole particles have a particle size of less than the effective average, by weight (or by other suitable measurement technique, such as by volume, number, *etc*.), *i.e.,* less than about 2000 nm, less than about 1900 nm, less than about 1800 nm, etc., when measured by techniques such as those noted above. In some embodiments, at least about 70%, about 90%, or about 95% of the aripiprazole particles have a particle size of less than the effective average, *i.e.,* less than about 2000 nm, 1900 nm, 1800 nm, 1700 nm, *etc.* In other embodiments, at least about 99% of the particles have a particle size less than the effective average particle size, *i.e*., les than about 2000 nm, less than about 1900 nm, less than about 1800 nm, less than about 1700 nm, *etc.*

As used herein, the value for D50 of a nanoparticulate aripiprazole composition is the particle size below which 50% of the aripiprazole particles fall, by weight (or by other suitable measurement technique, such as by volume, number, etc.). Similarly, D90 is the particle size below which 90% of the aripiprazole particles fall, by weight (or by other suitable measurement technique, such as by volume, number, etc.).

### 5. Concentration of Aripiprazole and Surface Stabilizers

The relative amounts of aripiprazole, or a salt or derivative thereof, and one or more surface stabilizers may vary. The optimal amount of the individual components can depend, for example, upon the particular aripiprazole selected, the hydrophilic lipophilic balance (HLB), melting point, and the surface tension of water solutions of the stabilizer, etc.

In some embodiments, the concentration of the aripiprazole may vary from about 99.5% to about 0.001%, from about 95% to about 0.1%, or from about 90% to about 0.5%, by weight, based on the total combined dry weight of the aripiprazole and at least one surface stabilizer, not including other excipients. In other embodiments, the compositions may include aripiprazole present in an amount of between about 5% to about 50% by weight.

In other embodiments, the concentration of the at least one surface stabilizer may vary from about 0.5% to about 99.999%, from about 5.0% to about 99.9%, or from about 10% to about 99.5%, by weight, based on the total combined dry weight of the aripiprazole and at least one surface stabilizer, not including other excipients. In other embodiments, the stabilizer may be present in an amount from about 0.1 % to about 50% by weight.

### 6. Injectable Nanoparticulate Aripiprazole Formulations

In some embodiments, injectable nanoparticulate aripiprazole formulations are provided. The following example is not intended to limit the scope of nanoparticulate injectable formulations in any respect, but rather to provide exemplary formulations which can be utilized as described herein and by methods known in the art. In some embodiments, the injectable formulations may comprise high drug concentrations in low injection volumes. Further, duration of action may be controlled via manipulation of particle size and hence dissolution, resulting in efficacious blood levels for extended periods; for example, greater than 2 days, greater than 5 days, greater than 7 days, greater than 10 days or greater than 14 days, one month, two months, three months or four months. An illustrative, non-limiting compositions is described below (based on % w/w):

| | |
|---|---|
| Aripiprazole | 5-50% |
| Stabilizer polymer | 0.1-50% |
| preservatives (Optional) | 0.05-0.25% |
| pH adjusting agent | pH about 6 to about 7 |
| water for injection | q.s. |

Exemplary preservatives include methylparaben (about 0.18% based on % w/w), propylparaben (about 0.02% based on % w/w), phenol (about 0.5% based on % w/w), and benzyl alcohol (up to 2% v/v). An exemplary pH adjusting agent is sodium hydroxide, and an exemplary liquid carrier is sterile water for injection. Other useful preservatives, pH adjusting agents, and liquid carriers are well-known in the art.

Exemplary surface stabilizers for injectable aripiprazole formulations may include but are not limited to stabilizers such as povidone polymer, hydroxypropyl cellulose, hydroxypropyl methyl cellulose, providone, polyvinyl pyrrolidone (PVP), pluronics, Tween®, peg-phospholipids and mixtures thereof. In some embodiments, stabilizers such as povidone, with a molecular weight of less than about 40,000 daltons, may be preferred. These stabilizers may be adsorbed onto the surface of the aripiprazole particle in an amount sufficient to maintain an effective average particle size for the desired duration of efficacy. Further, the nanoparticle size can be manipulated to give the desirable blood level profiles and duration of action when administered by either IM or SC routes.

### 7. Exemplary Nanoparticulate Aripiprazole Tablet Formulations

Several exemplary aripiprazole tablet formulations are given below. These examples are not intended to limit the scope of the invention in any respect, but rather to provide exemplary tablet formulations of aripiprazole which can be utilized as described herein and by methods known in the art. Such exemplary tablets can also comprise a coating agent.

| **Exemplary Nanoparticulate** **Aripiprazole Tablet Formulation #1** | |
|---|---|
| **Component** | **g/Kg** |
| Aripiprazole | about 50 to about 500 |
| Hypromellose, USP | about 10 to about 70 |
| Docusate Sodium, USP | about 1 to about 10 |
| Sucrose, NF | about 100 to about 500 |
| Sodium Lauryl Sulfate, NF | about 1 to about 40 |
| Lactose Monohydrate, NF | about 50 to about 400 |
| Silicified Microcrystalline Cellulose | about 50 to about 300 |
| Crospovidone, NF | about 20 to about 300 |
| Magnesium Stearate, NF | about 0.5 to about 5 |

| **Exemplary Nanoparticulate** **Aripiprazole Tablet Formulation #2** | |
|---|---|
| **Component** | **g/Kg** |
| Aripiprazole | about 100 to about 300 |
| Hypromellose, USP | about 30 to about 50 |
| Docusate Sodium, USP | about 0.5 to about 10 |
| Sucrose, NF | about 100 to about 300 |
| Sodium Lauryl Sulfate, NF | about 1 to about 30 |
| Lactose Monohydrate, NF | about 100 to about 300 |
| Silicified Microcrystalline Cellulose | about 50 to about 200 |
| Crospovidone, NF | about 50 to about 200 |
| Magnesium Stearate, NF | about 0.5 to about 5 |

| **Exemplary Nanoparticulate** **Aripiprazole Tablet Formulation #3** | |
|---|---|
| **Component** | **g/Kg** |
| Aripiprazole | about 200 to about 225 |
| Hypromellose, USP | about 42 to about 46 |
| Docusate Sodium, USP | about 2 to about 6 |
| Sucrose, NF | about 200 to about 225 |
| Sodium Lauryl Sulfate, NF | about 12 to about 18 |
| Lactose Monohydrate, NF | about 200 to about 205 |
| Silicified Microcrystalline Cellulose | about 130 to about 135 |
| Crospovidone, NF | about 112 to about 118 |
| Magnesium Stearate, NF | about 0.5 to about 3 |

| **Exemplary Nanoparticulate** **Aripiprazole Tablet Formulation #4** | |
|---|---|
| **Component** | **g/Kg** |
| Aripiprazole | about 119 to about 224 |
| Hypromellose, USP | about 42 to about 46 |
| Docusate Sodium, USP | about 2 to about 6 |
| Sucrose, NF | about 119 to about 224 |
| Sodium Lauryl Sulfate, NF | about 12 to about 18 |
| Lactose Monohydrate, NF | about 119 to about 224 |
| Silicified Microcrystalline Cellulose | about 129 to about 134 |
| Crospovidone, NF | about 112 to about 118 |
| Magnesium Stearate, NF | about 0.5 to about 3 |

### D. Methods of Making Nanoparticulate Aripiprazole Formulations

In another aspect of the invention includes method for preparing nanoparticulate aripiprazole formulations. The nanoparticulate aripiprazole, or a salt or derivative thereof, compositions can be made using, for example, milling, homogenization, precipitation, freezing, template emulsion techniques, or supercritical fluid techniques. Exemplary methods of making nanoparticulate compositions are described in the '684 patent, in U.S. Patent No. 5,518,187 for "Method of Grinding Pharmaceutical Substances"; U.S. Patent No. 5,718,388 for "Continuous Method of Grinding Pharmaceutical Substances"; U.S. Patent No. 5,862,999 for "Method of Grinding Pharmaceutical Substances"; U.S. Patent No. 5,665,331 for "Co-Microprecipitation of Nanoparticulate Pharmaceutical Agents with Crystal Growth Modifiers"; U.S. Patent No. 5,662,883 for "Co-Microprecipitation of Nanoparticulate Pharmaceutical Agents with Crystal Growth Modifiers"; U.S. Patent No. 5,560,932 for "Microprecipitation of Nanoparticulate Pharmaceutical Agents"; U.S. Patent No. 5,543,133 for "Process of Preparing X-Ray Contrast Compositions Containing Nanoparticles"; U.S. Patent No. 5,534,270 for "Method of Preparing Stable Drug Nanoparticles"; U.S. Patent No. 5,510,118 for "Process of Preparing Therapeutic Compositions Containing Nanoparticles"; and U.S. Patent No. 5,470,583 for "Method of Preparing Nanoparticle Compositions Containing Charged Phospholipids to Reduce Aggregation," all of which are specifically incorporated by reference.

The resultant nanoparticulate aripiprazole compositions may be utilized in injectable liquid dosage formulations, as a depot, as liquid dispersions, controlled release formulations, solid dosage formulations, lyophilized formulations, liquid dosage forms, as aerosols, ointments, creams, controlled release formulations, fast melt formulations, lyophilized formulations, tablets capsules, delayed release formulations, extended release formulations, pulsatile release formulations, mixed immediate release and controlled release formulations, etc.

### 1. Milling to Obtain Nanoparticulate Aripiprazole Dispersions

Milling aripiprazole, or a salt or derivative thereof, to obtain a nanoparticulate dispersion comprises dispersing the aripiprazole particles in a liquid dispersion medium in which the aripiprazole is poorly soluble, followed by applying mechanical means in the presence of grinding media to reduce the particle size of the aripiprazole to the desired effective average particle size. The dispersion medium can be, for example, water, safflower oil, ethanol, t-butanol, glycerin, polyethylene glycol (PEG), hexane, or glycol. In some embodiments, a preferred dispersion medium is water.

The aripiprazole particles may be reduced in size in the presence of at least one surface stabilizer, which may be added to the dispersion media before, during, or after particle size reduction. The liquid dispersion media may be maintained at a physiologic pH, for example, within a range of from about 3.0 to about 8.0 during the size reduction process; in some embodiments, the pH range may be more preferably within the range of from about 5.0 to about 7.5 during the size reduction process.

Other compounds, such as a diluent, can be added to the aripiprazole/surface stabilizer composition during the size reduction process. Dispersions can be manufactured continuously or in a batch mode.

By way of example, but not by way of limitation, a method of preparing an injectable nanoparticulate aripiprazole formulation may comprise: (1) dispersing aripiprazole in a liquid dispersion medium comprising a stabilizer such as but not limited to one or more of the following: a povidone polymer, hydroxypropyl cellulose, hydroxypropyl methyl cellulose, povidone, polyvinyl pyrrolidone (PVP), pluronics, Tween®, PEG-phospholipids and mixtures thereof with a molecular weight of less than about 40,000 daltons; and (2) mechanically reducing the particle size of the aripiprazole to an effective average particle size of less than about 1-2 µm. In some embodiments, the pH of the liquid dispersion medium may be maintained within the range of from about 3.0 to about 8.0 during the size reduction process; in other embodiments, the pH may be maintained at about 7.4.

### 2. Precipitation to Obtain Nanoparticulate Aripiprazole Compositions

Another method of forming the desired nanoparticulate aripiprazole compositions is by microprecipitation. This is a method of preparing stable dispersions of poorly soluble active agents in the presence of one or more surface stabilizers and one or more colloid stability enhancing surface active agents free of any trace toxic solvents or solubilized heavy metal impurities. Such a method may comprise, for example: (1) dissolving the aripiprazole in a suitable solvent; (2) adding the formulation from step (1) to a solution comprising at least one surface stabilizer; and (3) precipitating the formulation from step (2) using an appropriate non-solvent. The method can be followed by removal of any formed salt, if present, by dialysis or diafiltration and concentration of the dispersion by conventional means.

### 3. Homogenization to Obtain Nanoparticulate Aripiprazole Compositions

Exemplary homogenization methods of preparing active agent nanoparticulate compositions are described in U.S. Patent No. 5,510,118, for "Process of Preparing Therapeutic Compositions Containing Nanoparticles." Such a method comprises dispersing particles of an aripiprazole, or a salt or derivative thereof, in a liquid dispersion medium, followed by subjecting the dispersion to homogenization to reduce the particle size of an aripiprazole to the desired effective average particle size. The aripiprazole particles may be reduced in size in the presence of at least one surface stabilizer. Alternatively, the aripiprazole particles may be contacted with one or more surface stabilizers either before or after attrition. Other compounds, such as a diluent, can be added to the aripiprazole/surface stabilizer composition either before, during, or after the size reduction process. Dispersions can be manufactured continuously or in a batch mode.

### 4. Cryogenic Methodologies to Obtain Nanoparticulate Aripiprazole Compositions

Another method of forming the desired nanoparticulate aripiprazole compositions is by spray freezing into liquid ("SFL"). This technology comprises an organic or organoaqueous solution of aripiprazole with stabilizers, which is injected into a cryogenic liquid, such as liquid nitrogen. The droplets of the aripiprazole solution freeze at a rate sufficient to minimize crystallization and particle growth, thus formulating nanostructured aripiprazole particles. Depending on the choice of solvent system and processing conditions, the nanoparticulate aripiprazole particles can have varying particle morphology. In the isolation step, the nitrogen and solvent are removed under conditions that avoid agglomeration or ripening of the aripiprazole particles.

As a complementary technology to SFL, ultra rapid freezing ("URF") may also be used to created equivalent nanostructured aripiprazole particles with greatly enhanced surface area. URF comprises an organic or organoaqueous solution of aripiprazole with stabilizers onto a cryogenic substrate.

### 5. Emulsion Methodologies to Obtain Nanoparticulate Aripiprazole Compositions

Another method of forming the desired nanoparticulate aripiprazole, or a salt or derivative thereof, composition is by template emulsion. Template emulsion creates nanostructured aripiprazole particles with controlled particle size distribution and rapid dissolution performance. The method comprises an oil-in-water emulsion that is prepared, then swelled with a non-aqueous solution comprising the aripiprazole and stabilizers. The particle size distribution of the aripiprazole particles is a direct result of the size of the emulsion droplets prior to loading with the aripiprazole a property which can be controlled and optimized in this process. Furthermore, through selected use of solvents and stabilizers, emulsion stability is achieved with no or suppressed Ostwald ripening. Subsequently, the solvent and water are removed, and the stabilized nanostructured aripiprazole particles are recovered. Various aripiprazole particles morphologies can be achieved by appropriate control of processing conditions.

### 6. Supercritical Fluid Techniques Used to Obtain Nanoparticulate Aripiprazole Compositions

Published International Patent Application No. WO 97/144407 to Pace et al., published April 24, 1997, discloses particles of water insoluble biologically active compounds with an average size of 100 nm to 300 nm that are prepared by dissolving the compound in a solution and then spraying the solution into compressed gas, liquid or supercritical fluid in the presence of appropriate surface modifiers.

### 7. Sterile Product Manufacturing

Development of injectable compositions requires the production of a sterile product. The manufacturing process of the present invention is similar to typical known manufacturing processes for sterile suspensions. A typical sterile suspension manufacturing process flowchart is as follows:

| |
|---|
| (Media Conditioning) |
| ↓ |
| Compounding |
| ↓ |
| Particle Size Reduction |
| ↓ |
| Vial Filling |
| ↓ |
| (Lyophilization) and/or (Terminal Sterilization) |

As indicated by the optional steps in parentheses, some of the processing is dependent upon the method of particle size reduction and/or method of sterilization. For example, media conditioning is not required for a milling method that does not use media. If terminal sterilization is not feasible due to chemical and/or physical instability, aseptic processing can be used.

### E. Methods of Using the Nanoparticulate Aripiprazole Compositions of the Invention

Yet another aspect of the present invention provides methods of using the compositions described herein. The compositions of the invention are proposed to be useful in the treatment of diseases and disorders of the CNS, such as mental diseases and disorders, including but not limited to schizophrenia, acute manic and mixed episodes associated with bipolar disorder, and other schizophreniform illnesses. Thus, in some embodiments, the methods may include treating a mammal, including a human, for disorders of the central nervous system, such as mental diseases or disorders; such treatments may include psychiatric treatment. In some embodiments, treatment may involve administering to the mammal a composition comprising a nanoparticulate aripiprazole composition.

The compositions may be administered in any pharmaceutically acceptable form; however, in some embodiments, an injectable formulation may be preferred.

For example, the injectable formulation may be administered as an intramuscular or subcutaneous injection so as to form a bolus or depot; the depot may allow for a prolonged duration of action, for example, by dissolving slowly and steadily into the subject's system. Thus, the injectable formulations may be configured to allow for the controlled release of the aripiprazole after subcutaneous, intramuscular, intraperitoneal, etc. injection. For example, particle size and excipient concentration may be adjusted to result in the controlled release (*e.g*., the blood levels of aripiprazole in the subject's remain within an effective therapeutic window) for greater than 3 days, for greater than 5 days, for greater than 7 days, for greater than 10 days, for greater than 14 days, for greater than for 20 days, for greater than 30 days, for greater than 2 months, for greater than 3 months or for greater than 4 months. In some embodiments, the compositions may be formulated such that the injected depot may release aripiprazole at therapeutic levels for periods of from about two to about twenty-four weeks; from about two to about six weeks; from about two to about four weeks; and from about one to about four weeks.

In psychotropic therapy and the treatment of central nervous system disorders, it is useful to provide a drug dosage form that delivers the required therapeutic amount of the drug in vivo and renders the drug bioavailable in a rapid and consistent manner. These goals may be achieved using the injectable nanoparticulate formulations, such as aripiprazole, described herein, via the formation of a depot (*e.g*., with intramuscular injection) as described above. In some embodiments, the drug is released from the depot into the blood stream at a constant rate, thus providing the patient with the proper dose of the drug continuously for an extended period of time. This method (*e.g*., depot injection) also results in improved patient compliance. A single injection once per month, for example, will provide the patient with the appropriate therapeutic dosage for the month, versus the daily struggle to remember or to decide to take a tablet, capsule, etc.

An exemplary injectable formulation of aripiprazole for intramuscular or subcutaneous administration may include nanoparticulate aripiprazole having one or more stabilizers, such as but not limited to, a povidone polymer, hydroxypropyl cellulose, hydroxypropyl methyl cellulose, providone, polyvinyl pyrrolidone (PVP), pluronics, Tween®, PEG-phospholipids and mixtures thereof, with a molecular weight of less than about 40,000 daltons adsorbed on the surface thereof in an amount sufficient to maintain an effective average particle size for the desired duration of efficacy. Such aripiprazole compositions formulated for parenteral administration may eliminate the need for toxic co-solvents and enhance the efficacy of aripiprazole in the treatment of various types CNS diseases or disorders, such as mental diseases and disorders.

Compositions suitable for parenteral injection may comprise physiologically acceptable sterile aqueous or nonaqueous solutions, dispersions, suspensions or emulsions, and sterile powders for reconstitution into sterile injectable solutions or dispersions. Examples of suitable aqueous and nonaqueous carriers, diluents, solvents, or vehicles including water, ethanol, polyols (propyleneglycol, polyethyleneglycol, glycerol, and the like), suitable mixtures thereof, vegetable oils (such as olive oil) and injectable organic esters such as ethyl oleate. Proper fluidity can be maintained, for example, by the use of a coating such as lecithin, by the maintenance of the required particle size in the case of dispersions, and by the use of surfactants.

The nanoparticulate aripiprazole, or a salt or derivative thereof, compositions may also contain adjuvants such as preserving, wetting, emulsifying, and dispensing agents. Prevention of the growth of microorganisms can be ensured by various antibacterial and antifungal agents, such as parabens, chlorobutanol, phenol, sorbic acid, and the like. It may also be desirable to include isotonic agents, such as sugars, sodium chloride, and the like. Prolonged absorption of the injectable pharmaceutical form can be brought about by the use of agents delaying absorption, such as aluminum monostearate and gelatin.

In addition, it is anticipated that a higher concentration of the nanoparticulate form of aripiprazole may be delivered in a smaller injectable dose size (and thus smaller volume) as compared to conventional forms of aripiprazole. Accordingly, the subject is anticipated to experience minimal or no discomfort or irritation after injection of nanoparticulate aripiprazole formulations, as compared with the injection of conventional formulations.

Solid dosage forms for oral administration are also contemplated and include, but are not limited to, capsules, tablets, pills, powders, and granules. In such solid dosage forms, the active agent is admixed with at least one of the following: (a) one or more inert excipients (or carriers), such as sodium citrate or dicalcium phosphate; (b) fillers or extenders, such as starches, lactose, sucrose, glucose, mannitol, and silicic acid; (c) binders, such as carboxymethylcellulose, alignates, gelatin, polyvinylpyrrolidone, sucrose, and acacia; (d) humectants, such as glycerol; (e) disintegrating agents, such as agar-agar, calcium carbonate, potato or tapioca starch, alginic acid, certain complex silicates, and sodium carbonate; (f) solution retarders, such as paraffin; (g) absorption accelerators, such as quaternary ammonium compounds; (h) wetting agents, such as cetyl alcohol and glycerol monostearate; (i) adsorbents, such as kaolin and bentonite; and (j) lubricants, such as talc, calcium stearate, magnesium stearate, solid polyethylene glycols, sodium lauryl sulfate, or mixtures thereof. For capsules, tablets, and pills, the dosage forms may also comprise buffering agents.

Liquid dosage forms for oral administration may include pharmaceutically acceptable emulsions, solutions, suspensions, syrups, and elixirs. In addition to an aripiprazole, the liquid dosage forms may comprise inert diluents commonly used in the art, such as water or other solvents, solubilizing agents, and emulsifiers. Exemplary emulsifiers are ethyl alcohol, isopropyl alcohol, ethyl carbonate, ethyl acetate, benzyl alcohol, benzyl benzoate, propyleneglycol, 1,3-butyleneglycol, dimethylformamide, oils, such as cottonseed oil, groundnut oil, corn germ oil, olive oil, castor oil, and sesame oil, glycerol, tetrahydrofurfuryl alcohol, polyethyleneglycols, fatty acid esters of sorbitan, or mixtures of these substances, and the like.

Besides such inert diluents, the composition can also include adjuvants, such as wetting agents, emulsifying and suspending agents, sweetening, flavoring, and perfuming agents.

The present disclosure also provides methods of rapidly increasing the bioavailability (*e.g*., plasma levels) of aripiprazole in a subject. By way of example, but not by way of limitation, such methods may include parenterally or orally administering to a subject an effective amount of a composition comprising a nanoparticulate aripiprazole. For example, in some embodiments, the aripiprazole compositions may be administered orally and, in accordance with standard pharmacokinetic practice, the compositions may have a bioavailability that is about 50% greater, about 40% greater, about 30% greater, about 20% greater or about 10% greater than a conventional dosage form. Additionally, when tested in fasting subjects in accordance with standard pharmacokinetic practice, the nanoparticulate aripiprazole compositions may produce a maximum blood plasma concentration profile in less than about 6 hours, less than about 5 hours, less than about 4 hours, less than about 3 hours, less than about 2 hours, less than about 1 hour, or less than about 30 minutes after the initial dose of the compositions.

Though injectable compositions may be preferred in certain embodiments, the aripiprazole compounds are contemplated to be administered to a subject via any conventional means including, but not limited to, orally, rectally, ocularly, parenterally (*e.g*., intravenous, intramuscular, intraperitoneal or subcutaneous), intracisternally, pulmonary, intravaginally, intraperitoneally, locally (*e.g*., powders, ointments or drops), as a bioadhesive, or as a buccal or nasal spray.

As used herein, the term "subject" is used to mean an animal, preferably a mammal, including a human or non-human. The terms patient and subject may be used interchangeably.

"Therapeutically effective amount" as used herein with respect to an aripiprazole, dosage shall mean that dosage that provides the specific pharmacological response for which an aripiprazole is administered in a significant number of subjects in need of such treatment. It is emphasized that "therapeutically effective amount," administered to a particular subject in a particular instance will not always be effective in treating the diseases described herein, even though such dosage is deemed a "therapeutically effective amount"' by those skilled in the art. It is to be further understood that aripiprazole dosages are, in particular instances, measured as oral dosages, or with reference to drug levels as measured in blood.

One of ordinary skill will appreciate that effective amounts of an aripiprazole can be determined empirically and can be employed in pure form or, where such forms exist, in pharmaceutically acceptable salt, ester, or prodrug form. Actual dosage levels of an aripiprazole in the nanoparticulate compositions of the invention may be varied to obtain an amount of an aripiprazole that is effective to obtain a desired therapeutic response for a particular composition and method of administration. The selected dosage level therefore depends upon the desired therapeutic effect, the route of administration, the potency of the administered aripiprazole, the desired duration of treatment, and other factors.

Dosage unit compositions may contain such amounts of such submultiples thereof as may be used to make up the daily dose. It will be understood, however, that the specific dose level for any particular patient will depend upon a variety of factors: the type and degree of the cellular or physiological response to be achieved; activity of the specific agent or composition employed; the specific agents or composition employed; the age, body weight, general health, sex, and diet of the patient; the time of administration, route of administration, and rate of excretion of the agent; the duration of the treatment; drugs used in combination or coincidental with the specific agent; and like factors well known in the medical arts.

### EXAMPLES

The following examples are given to illustrate the present invention. It should be understood, however, that the spirit and scope of the invention is not to be limited to the specific conditions or details described in these examples.

### Example 1

The purpose of this example is to illustrate the procedure for identifying a suitable nanoparticulate formulation of aripiprazole.

The study can be conducted by screening a number of surface stabilizers (here, seventeen different surface stabilizers and combinations of stabilizers have been selected) to identify the most suitable stabilizer for a particular administration format, such as here, for the parenteral administration of nanoparticulate aripiprazole.

The following examples are based on an aqueous dispersion of 5% (w/w) aripiprazole, combined with the exemplary surface stabilizers. Table 2 provides exemplary weight percentages of particular surface stabilizers; deionized water would be used to make up the weight percent to 100%. Table 3 lists additional preferred stabilizers. Such formulations could be milled in a 10-ml chamber of a NanoMill® 0.01 (NanoMill Systems, King of Prussion, PA; *see e.g.,* U.S. Patent No, 6,431,478), along with 500 micron PolyMill® attrition media (Dow Chemical Co.) (*e.g*., at 89% media load). The dispersions could be formulated at 40% solids to 2.4% surface stabilizer. In an exemplary process, the mixtures could be milled at a speed of 2500-3500 rpm for 30- 90 minutes, (for example, 60 minutes at 2500 rpm); optimal milling speed and milling time may be determined empirically for any given formulation.

Following milling, the particle size of the milled aripiprazole particles could be measured, in deionized, distilled water, using a Horiba LA 910 particle size analyzer. Additionally or alternatively, particles may be evaluated using a Lecia DM5000B microscope and Lecia CTR 5000 light source (Laboratory Instruments & Supplies (I) Ltd. Ashbourne CO MEATH ROI). For a successful composition, the initial mean and/or D50 milled aripiprazole particle size would be expected to be less than about 2000 nm. Particle size could also be evaluated after sonication for varying times, for example, after sonication for 30, 60 or 90 seconds. For successful compositions, the initial mean and/or D50 milled particle size would be expected to be less than about 2000 nm.

**TABLE 2**

| **No.** | **Surface Stabilizer (percent by weight)** |
|---|---|
| 1 | Hydroxy propyl methyl cellulose ("HPMC") 1.25%; dioctylsulfosuccinate ("DOSS") 0.05% |
| 2 | Hydroxypropyl cellulose ("HPC") (super-low viscosity) 1.25%; DOSS 0.05% |
| 3 | HPC (super-low viscosity) 1.25%; Sodium lauryl sulphate 0.05% |
| 4 | Plasdone® S-630 1.25%; DOSS 0.05% |
| 5 | Polyvinylpyrrolodone ("PVP") C15 1.25%; Dioxycholic acid 0.05% |

**TABLE 3**

| **No.** | **Other Preferred Surface Stabilizer** |
|---|---|
| 1 | Plasdone C15^{®} (polyvinylpyrrolidone) |
| 2 | Kollidon 17PF^{®} (a polyvinylpyrrolidone polymer) |
| 3 | Povidone K30^{®} (a polyvinylpyrrolidone polymer) |
| 4 | Tyloxapol |
| 5 | Pluronic F68^{®} (a high molecular weight polyoxyalkylene ether) |
| 6 | PluronicF108^{®} (a high molecular weight polyoxyalkylene ether) |
| 7 | Tween 80^{®} (a polyoxyethylene sorbitan fatty acid ester) |
| 8 | dioctylsulfosuccinate (CAS No. 577-11-7; aka Docusate Sodium) |
| 9 | B20-5000^{®} (a triblock copolymer surface modifier) |
| 10 | B20-5000-sulfonated (a triblock copolymer surface modifier) |
| 11 | lecithin (CAS No. 8002-43-5) |
| 12 | Povidone K30^{®} and Pluronic F108^{®} |

Such combinations may produce stable dispersions of differing nanoparticulate size that will have differing durations of action when administered. Preclinical and clinical studies could be used to identify the optimum formulation and size associated with the desired prolonged duration of action.

It will be apparent to those skilled in the art that various modifications and variations can be made in the methods and compositions of the present inventions without departing from the spirit or scope of the invention. Thus, it is intended that the present invention cover the modification and variations of the invention provided they come within the scope of the appended claims and their equivalents.

The terms and expressions which have been employed are used as terms of description and not of limitation, and there is no intention that in the use of such terms and expressions of excluding any equivalents of the features shown and described or portions thereof, but it is recognized that various modifications are possible within the scope of the invention. Thus, it should be understood that although the present invention has been illustrated by specific embodiments and optional features, modification and/or variation of the concepts herein disclosed may be resorted to by those skilled in the art, and that such modifications and variations are considered to be within the scope of this invention.

In addition, where features or aspects of the invention are described in terms of Markush groups or other grouping of alternatives, those skilled in the art will recognize that the invention is also thereby described in terms of any individual member or subgroup of members of the Markush group or other group.

Also, unless indicated to the contrary, where various numerical values are provided for embodiments, additional embodiments are described by taking any 2 different values as the endpoints of a range. Such ranges are also within the scope of the described invention.

All references, patents, and/or applications cited in the specification are incorporated by reference in their entireties, including any tables and figures, to the same extent as if each reference had been incorporated by reference in its entirety individually.

The following pages 51 to 59 contain preferred embodiments. Accordingly, the term "claim" as used therein refers to such a "preferred embodiment".
1. A stable nanoparticulate aripiprazole, or salt or derivative thereof, composition comprising:
   (a) particles of aripiprazole having an average effective particle size of less than about 2000 nm; and
   (b) at least one surface stabilizer.
2. The composition of claim 1, wherein the aripiprazole is in a form selected from the group consisting of a crystalline phase, an amorphous phase, a semi-crystalline phase, a semi amorphous phase, and mixtures thereof.
3. The composition of claim 1, wherein the effective average particle size of the aripiprazole particles is selected from the group consisting of less than about 1900 nm, less than about 1800 nm, less than about 1700 nm, less than about 1600 nm, less than about 1500 nm, less than about 1400 nm, less than about 1300 nm, less than about 1200 nm, less than about 1100 nm, less than about 1000 nm, less than about 900 nm, less than about 800 nm, less than about 700 nm, less than about 600 nm, less than about 500 nm, less than about 400 nm, less than about 300 nm, less than about 250 nm, less than about 200 nm, less than about 100 nm, less than about 75 nm, and less than about 50 nm.
4. The composition of claim 1, wherein the composition is formulated:
   (a) for administration selected from the group consisting of parenteral, oral, pulmonary, intravenous, rectal, ophthalmic, colonic, intracisternal, intravaginal, intraperitoneal, ocular, otic, local, buccal, nasal, bioadhesive and topical administration;
   (b) into a dosage form selected from the group consisting of liquid dispersions, gels, aerosols, ointments, creams, lyophilized formulations, tablets, capsules;
   (c) into a dosage form selected from the group consisting of controlled release formulations, fast melt formulations, delayed release formulations, extended release formulations, pulsatile release formulations, mixed immediate release formulations, controlled release formulations; or
   (d) any combination of (a), (b), and (c).
5. The composition of claim 1, wherein the composition further comprises one or more pharmaceutically acceptable excipients, carriers or a combination thereof.
6. The composition of claim 1, wherein
   (a) the amount of aripiprazole is selected from the group consisting of from about 99.5% to about 0.001%, from about 95% to about 0.1%, and from about 90% to about 0.5%, by weight, based on the total combined weight of aripiprazole and at least one surface stabilizer, not including other excipients;
   (b) at least one surface stabilizer is present in an amount selected from the group consisting of from about 0.5% to about 99.999% by weight, from about 5.0% to about 99.9% by weight, and from about 10% to about 99.5% by weight, based on the total combined dry weight of aripiprazole and at least one surface stabilizer, not including other excipients; or
   (c) a combination of (a) and (b).
7. The composition of claim 1, further comprising at least one primary surface stabilizer and at least one secondary surface stabilizer.
8. The composition of claim 1, wherein the surface stabilizer is selected from the group consisting of an anionic surface stabilizer, a cationic surface stabilizer, a zwitterionic surface stabilizer, a non-ionic surface stabilizer, and an ionic surface stabilizer.
9. The composition of claim 1, wherein at least one surface stabilizer is selected from the group consisting of cetyl pyridinium chloride, gelatin, casein, phosphatides, dextran, glycerol, gum acacia, cholesterol, tragacanth, stearic acid, benzalkonium chloride, calcium stearate, glycerol monostearate, cetostearyl alcohol, cetomacrogol emulsifying wax, sorbitan esters, polyoxyethylene alkyl ethers, polyoxyethylene castor oil derivatives, polyoxyethylene sorbitan fatty acid esters, polyethylene glycols, dodecyl trimethyl ammonium bromide, polyoxyethylene stearates, colloidal silicon dioxide, phosphates, sodium dodecylsulfate, carboxymethylcellulose calcium, hydroxypropyl celluloses, hypromellose, carboxymethylcellulose sodium, methylcellulose, hydroxyethylcellulose, hypromellose phthalate, noncrystalline cellulose, magnesium aluminum silicate, triethanolamine, polyvinyl alcohol, polyvinylpyrrolidone, 4-(1,1,3,3-tetramethylbutyl)-phenol polymer with ethylene oxide and formaldehyde, poloxamers; poloxamines, a charged phospholipid, dioctylsulfosuccinate (dioctyl sodium sulfosuccinate), dialkylesters of sodium sulfosuccinic acid, sodium lauryl sulfate, alkyl aryl polyether sulfonates, mixtures of sucrose stearate and sucrose distearate, C₁₈H₃₇CH₂C(O)N(CH₃)-CH₂(CHOH)₄(CH₂OH)₂, p-isononylphenoxypoly-(glycidol), decanoyl-N-methylglucamide; n-decyl β-D-glucopyranoside; n-decyl β-D-maltopyranoside; n-dodecyl β-D-glucopyranoside; n-dodecyl β-D-maltoside; heptanoyl-N-methylglucamide; n-heptyl-β-D-glucopyranoside; n-heptyl β-D-thioglucoside; n-hexyl β-D-glucopyranoside; nonanoyl-N-methylglucamide; n-noyl β-D-glucopyranoside; octanoyl-N-methylglucamide; n-octyl-β-D-glucopyranoside; octyl β-D-thioglucopyranoside; lysozyme, PEG-phospholipid, PEG-cholesterol, PEG-cholesterol derivative, PEG-vitamin A, PEG-vitamin E, lysozyme, random copolymers of vinyl acetate and vinyl pyrrolidone, a cationic polymer, a cationic biopolymer, a cationic polysaccharide, a cationic cellulosic, a cationic alginate, a cationic nonpolymeric compound, cationic phospholipids, cationic lipids, polymethylmethacrylate trimethylammonium bromide, sulfonium compounds, polyvinylpyrrolidone-2-dimethylaminoethyl methacrylate dimethyl sulfate, hexadecyltrimethyl ammonium bromide, phosphonium compounds, quarternary ammonium compounds, benzyl-di(2-chloroethyl)ethylammonium bromide, coconut trimethyl ammonium chloride, coconut trimethyl ammonium bromide, coconut methyl dihydroxyethyl ammonium chloride, coconut methyl dihydroxyethyl ammonium bromide, decyl triethyl ammonium chloride, decyl dimethyl hydroxyethyl ammonium chloride, decyl dimethyl hydroxyethyl ammonium chloride bromide, C₁₂₋₁₅dimethyl hydroxyethyl ammonium chloride, C₁₂₋₁₅dimethyl hydroxyethyl ammonium chloride bromide, coconut dimethyl hydroxyethyl ammonium chloride, coconut dimethyl hydroxyethyl ammonium bromide, myristyl trimethyl ammonium methyl sulphate, lauryl dimethyl benzyl ammonium chloride, lauryl dimethyl benzyl ammonium bromide, lauryl dimethyl (ethenoxy)₄ ammonium chloride, lauryl dimethyl (ethenoxy)₄ ammonium bromide, N-alkyl (C₁₂₋₁₈)dimethylbenzyl ammonium chloride, N-alkyl (C₁₄₋₁₈)dimethyl-benzyl ammonium chloride, N-tetradecylidmethylbenzyl ammonium chloride monohydrate, dimethyl didecyl ammonium chloride, N-alkyl and (C₁₂₋₁₄) dimethyl 1-napthylmethyl ammonium chloride, trimethylammonium halide, alkyl-trimethylammonium salts, dialkyl-dimethylammonium salts, lauryl trimethyl ammonium chloride, ethoxylated alkyamidoalkyldialkylammonium salt, an ethoxylated trialkyl ammonium salt, dialkylbenzene dialkylammonium chloride, N-didecyldimethyl ammonium chloride, N-tetradecyldimethylbenzyl ammonium, chloride monohydrate, N-alkyl(C₁₂₋₁₄) dimethyl 1-naphthylmethyl ammonium chloride, dodecyldimethylbenzyl ammonium chloride, dialkyl benzenealkyl ammonium chloride, lauryl trimethyl ammonium chloride, alkylbenzyl methyl ammonium chloride, alkyl benzyl dimethyl ammonium bromide, C₁₂ trimethyl ammonium bromides, C₁₅ trimethyl ammonium bromides, C₁₇ trimethyl ammonium bromides, dodecylbenzyl triethyl ammonium chloride, polydiallyldimethylammonium chloride (DADMAC), dimethyl ammonium chlorides, alkyldimethylammonium halogenides, tricetyl methyl ammonium chloride, decyltrimethylammonium bromide, dodecyltriethylammonium bromide, tetradecyltrimethylammonium bromide, methyl trioctylammonium chloride, POLYQUAT 10™, tetrabutylammonium bromide, benzyl trimethylammonium bromide, choline esters, benzalkonium chloride, stearalkonium chloride compounds, cetyl pyridinium bromide, cetyl pyridinium chloride, halide salts of quaternized polyoxyethylalkylamines, MIRAPOL™, ALKAQUAT™, alkyl pyridinium salts; amines, amine salts, amine oxides, imide azolinium salts, protonated quaternary acrylamides, methylated quaternary polymers, and cationic guar.
10. The composition of claim 1, wherein at least one surface stabilizer is selected from the group consisting of povidone, povidone polymer, Plasdone®, hydroxypropyl cellulose, hydroxypropyl methyl cellulose, polyvinyl pyrrolidone, polyvinyl pyrrolidone polymers, high molecular weight polyoxyalkylene ethers, Pluronic™, polyoxyethylene sorbitan fatty acid esters, polyethylene glycol phospholipids, dioxycholic acid, dioctylsulfosuccinate, sodium laurel sulphate, triblock copolymer surface modifiers such as B20-5000® and B20-5000-sulfonated, tyloxapol, and lecithin.
11. The composition of claim 1 wherein the composition is formulated for subcutaneous or intramuscular injection.
12. The composition of claim 11, wherein the composition is formulated so as to form a depot upon injection.
13. The composition of claim 1, wherein the pharmacokinetic profile of said composition is not significantly affected by the fed or fasted state of a subject ingesting said composition.
14. The composition of claim 1, wherein the composition does not produce significantly different absorption levels when administered under fed as compared to fasting conditions.
15. The composition of claim 14, wherein the difference in absorption of the active agent composition of the invention, when administered in the fed versus the fasted state, is selected from the group consisting of less than about 100%, less than about 90%, less than about 80%, less than about 70%, less than about 60%, less than about 50%, less than about 40%, less than about 30%, less than about 25%, less than about 20%, less than about 15%, less than about 10%, less than about 5%, and less than about 3%.
16. The composition of claim 1, wherein administration of the composition to a subject in a fasted state is bioequivalent to administration of said composition to a subject in a fed state.
17. The composition of claim 16, wherein "bioequivalency" is established by:
   (a) a 90% Confidence Interval of between 0.80 and 1.25 for both Cₘₐₓ and AUC; or
   (b) a 90% Confidence Interval of between 0.80 and 1.25 for AUC and a 90% Confidence Interval of between 0.70 to 1.43 for Cₘₐₓ.
18. The composition of claim 1, wherein:
   (a) the Tₘₐₓ of the aripiprazole, when assayed in the plasma of a mammalian subject following administration, is less than the Tₘₐₓ for a non-nanoparticulate composition of the same aripiprazole, administered at the same dosage;
   (b) the Cₘₐₓ of the aripiprazole, when assayed in the plasma of a mammalian subject following administration, is greater than the Cₘₐₓ for a non-nanoparticulate composition of the same aripiprazole, administered at the same dosage;
   (c) the AUC of the aripiprazole, when assayed in the plasma of a mammalian subject following administration, is greater than the AUC for a non-nanoparticulate composition of the same aripiprazole, administered at the same dosage; or
   (d) any combination of (a), (b), and (c).
19. The composition of claim 18, wherein:
   (a) the Tₘₐₓ is selected from the group consisting of not greater than about 90%, not greater than about 80%, not greater than about 70%, not greater than about 60%, not greater than about 50%, not greater than about 30%, not greater than about 25%, not greater than about 20%, not greater than about 15%, not greater than about 10%, and not greater than about 5% of the Tₘₐₓ exhibited by a non-nanoparticulate composition of the same aripiprazole, administered at the same dosage;
   (b) the Cₘₐₓ is selected from the group consisting of at least about 50%, at least about 100%, at least about 200%, at least about 300%, at least about 400%, at least about 500%, at least about 600%, at least about 700%, at least about 800%, at least about 900%, at least about 1000%, at least about 1100%, at least about 1200%, at least about 1300%, at least about 1400%, at least about 1500%, at least about 1600%, at least about 1700%, at least about 1800%, or at least about 1900% greater than the Cₘₐₓ exhibited by a non-nanoparticulate composition of the same aripiprazole, administered at the same dosage;
   (c) the AUC is selected from the group consisting of at least about 25%, at least about 50%, at least about 75%, at least about 100%, at least about 125%, at least about 150%, at least about 175%, at least about 200%, at least about 225%, at least about 250%, at least about 275%, at least about 300%, at least about 350%, at least about 400%, at least about 450%, at least about 500%, at least about 550%, at least about 600%, at least about 750%, at least about 700%, at least about 750%, at least about 800%, at least about 850%, at least about 900%, at least about 950%, at least about 1000%, at least about 1050%, at least about 1100%, at least about 1150%, or at least about 1200% greater than the AUC exhibited by the non-nanoparticulate formulation of the same aripiprazole, administered at the same dosage; or
   (d) any combination of (a), (b), and (c).
20. The composition of claim 1, additionally comprising one or more active agents useful for the treatment of mental diseases or disorders.
21. The composition of claim 20, wherein said mental disease or disorder is selected from the group consisting of schizophreniform illness, schizophrenia, bipolar disorder and combinations thereof.
22. The composition of claim 20, wherein said one or more active agents is selected from the group consisting of chlorpromazine, fluphenazine, perphenazine and prochlorperazine, clozapine, olanzapine, quetiapine, ziprasidone and combinations thereof.
23. The composition claim 1, wherein:
   (a) upon administration to a mammal the aripiprazole particles redisperse such that the particles have an effective average particle size selected from the group consisting of less than about 2 microns, less than about 1900 nm, less than about 1800 nm, less than about 1700 nm, less than about 1600 nm, less than about 1500 nm, less than about 1400 nm, less than about 1300 nm, less than about 1200 nm, less than about 1100 nm, less than about 1000 nm, less than about 900 nm, less than about 800 nm, less than about 700 nm, less than about 600 nm, less than about 500 nm, less than about 400 nm, less than about 300 nm, less than about 250 nm, less than about 200 nm, less than about 150 nm, less than about 100 nm, less than about 75 nm, and less than about 50 nm;
   (b) the composition redisperses in a biorelevant media such that the aripiprazole particles have an effective average particle size selected from the group consisting of less than about 2 microns, less than about 1900 nm, less than about 1800 nm, less than about 1700 nm, less than about 1600 nm, less than about 1500 nm, less than about 1400 nm, less than about 1300 nm, less than about 1200 nm, less than about 1100 nm, less than about 1000 nm, less than about 900 nm, less than about 800 nm, less than about 700 nm, less than about 600 nm, less than about 500 nm, less than about 400 nm, less than about 300 nm, less than about 250 nm, less than about 200 nm, less than about 150 nm, less than about 100 nm, less than about 75 nm, and less than about 50 nm; or
   (c) a combination of (a) and (b).
24. A method of preparing a nanoparticulate aripiprazole, or a salt or derivative thereof, composition comprising: contacting particles of aripiprazole with at least one surface stabilizer for a time and under conditions sufficient to provide a nanoparticulate aripiprazole composition having an effective average particle size of less than about 2000 nm.
25. The method of claim 22, wherein contacting comprises a method selected from the group consisting of milling, homogenization, freezing, emulsion techniques, supercritical fluid particle generation techniques, precipitation, and combinations thereof.
26. A method for the treatment of schizophrenia, bipolar disorder, schizophreniform illnesses and related conditions in a subject comprising:
   administering to a subject of an effective amount of a composition comprising,
      (a) particles of aripiprazole or salt or derivative thereof having an average effective particle size of less than about 2000 nm; and
      (b) at least one surface stabilizer.
27. The method of claim 26, further comprising one or more active agents useful for the treatment of schizophrenia, bipolar disorder and related condition.
28. The method of claim 27, wherein the related condition is selected from the group consisting of extrapyramidal symptoms, drug-induced Parkinsonism, acute dystonic reactions, akathisia, tardiv dyskinesia, tardive distonia and a combination thereof.
29. The composition of claim 26, wherein the composition is formulated for subcutaneous or intramuscular injection.
30. The method of claim 26, wherein the composition is in the form of an oral tablet.

## Claims

1. A stable nanoparticulate aripiprazole, or salt thereof, parenteral composition comprising:
(a) particles of aripiprazole having an average effective particle size of less than about 2000 nm; and
(b) at least one surface stabilizer.

2. The composition of claim 1, wherein the aripiprazole is in a form selected from the group consisting of a crystalline phase, an amorphous phase, a semi- crystalline phase, and mixtures thereof.

3. The composition of any preceding claim, wherein the effective average particle size of the aripiprazole particles is selected from the group consisting of less than about 1900 nm, less than about 1800 nm, less than about 1700 nm, less than about 1600 nm, less than about 1500 nm, less than about 1400 nm, less than about 1300 nm, less than about 1200 nm, less than about 1100 nm, less than about 1000 nm, less than about 900 nm, less than about 800 nm, less than about 700 nm, less than about 600 nm, less than about 500 nm, less than about 400 nm, less than about 300 nm, less than about 250 nm, less than about 200 nm, less than about 100 nm, less than about 75 nm, and less than about 50 nm.

4. The composition of any preceding claim, wherein the composition is formulated:
(a) into a dosage form selected from the group consisting of liquid dispersions, gels, aerosols, lyophilized formulations;
(b) into a dosage form selected from the group consisting of controlled release formulations, delayed release formulations, extended release formulations, pulsatile release formulations, mixed immediate release formulations, controlled release formulations; or
(c) any combination of (a) and (b).

5. The composition of any preceding claim, wherein
(a) the amount of aripiprazole is selected from the group consisting of from about 99.5% to about 0.001%, from about 95% to about 0.1%, and from about 90% to about 0.5%, by weight, based on the total combined weight of aripiprazole and at least one surface stabilizer, not including other excipients;
(b) at least one surface stabilizer is present in an amount selected from the group consisting of from about 0.5% to about 99.999% by weight, from about 5.0% to about 99.9% by weight, and from about 10% to about 99.5% by weight, based on the total combined dry weight of aripiprazole and at least one surface stabilizer, not including other excipients; or
(c) a combination of (a) and (b).

6. The composition of any preceding claim, further comprising at least one primary surface stabilizer and at least one secondary surface stabilizer.

7. The composition of any preceding claim, wherein at least one surface stabilizer is selected from the group consisting of cetyl pyridinium chloride, gelatin, casein, phosphatides, dextran, glycerol, gum acacia, cholesterol, tragacanth, stearic acid, benzalkonium chloride, calcium stearate, glycerol monostearate, cetostearyl alcohol, cetomacrogol emulsifying wax, sorbitan esters, polyoxyethylene alkyl ethers, polyoxyethylene castor oil derivatives, polyoxyethylene sorbitan fatty acid esters, polyethylene glycols, dodecyl trimethyl ammonium bromide, polyoxyethylene stearates, colloidal silicon dioxide, phosphates, sodium dodecylsulfate, carboxymethylcellulose calcium, hydroxypropyl celluloses, hypromellose, carboxymethylcellulose sodium, methylcellulose, hydroxyethylcellulose, hypromellose phthalate, noncrystalline cellulose, magnesium aluminum silicate, triethanolamine, polyvinyl alcohol, polyvinylpyrrolidone, 4-(1,1,3,3-tetramethylbutyl)-phenol polymer with ethylene oxide and formaldehyde, poloxamers; poloxamines, a charged phospholipid, dioctylsulfosuccinate (dioctyl sodium sulfosuccinate), dialkylesters of sodium sulfosuccinic acid, sodium lauryl sulfate, alkyl aryl polyether sulfonates, mixtures of sucrose stearate and sucrose distearate, C₁₈H₃₇CH₂C(O)N(CH₃)- CH₂(CHOH)₄(CH₂OH)₂, p-isononylphenoxypoly-(glycidol), decanoyl-N-methylglucamide; n-decyl β -D-glucopyranoside; n-decyl β -D-maltopyranoside; n- dodecyl β-D-glucopyranoside; n-dodecyl β -D-maltoside; heptanoyl-N- methylglucamide; n-heptyl- β -D-glucopyranoside; n-heptyl β -D-thioglucoside; n- hexyl β -D-glucopyranoside; nonanoyl-N-methylglucamide; n-noyl β -D- glucopyranoside; octanoyl-N-methylglucamide; n-octyl- β -D-glucopyranoside; octyl β -D-thioglucopyranoside; lysozyme, PEG-phospholipid, PEG-cholesterol, PEG- cholesterol derivative, PEG-vitamin A, PEG-vitamin E, lysozyme, random copolymers of vinyl acetate and vinyl pyrrolidone, a cationic polymer, a cationic biopolymer, a cationic polysaccharide, a cationic cellulosic, a cationic alginate, a cationic nonpolymeric compound, cationic phospholipids, cationic lipids, polymethylmethacrylate trimethylammonium bromide, sulfonium compounds, polyvinylpyrrolidone-2-dimethylaminoethyl methacrylate dimethyl sulfate, hexadecyltrimethyl ammonium bromide, phosphonium compounds, quarternary ammonium compounds, benzyl-di(2-chloroethyl)ethylammonium bromide, coconut trimethyl ammonium chloride, coconut trimethyl ammonium bromide, coconut methyl dihydroxyethyl ammonium chloride, coconut methyl dihydroxyethyl ammonium bromide, decyl triethyl ammonium chloride, decyl dimethyl hydroxyethyl ammonium chloride, decyl dimethyl hydroxyethyl ammonium chloride bromide, C₁₂₋₁₅dimethyl hydroxyethyl ammonium chloride, Cn-isdimethyl hydroxyethyl ammonium chloride bromide, coconut dimethyl hydroxyethyl ammonium chloride, coconut dimethyl hydroxyethyl ammonium bromide, myristyl trimethyl ammonium methyl sulphate, lauryl dimethyl benzyl ammonium chloride, lauryl dimethyl benzyl ammonium bromide, lauryl dimethyl (ethenoxy)4 ammonium chloride, lauryl dimethyl (ethenoxy)4 ammonium bromide, N-alkyl (C₁₂₋₁₈)dimethylbenzyl ammonium chloride, N-alkyl (C₁₄₋₁₈)dimethyl-benzyl ammonium chloride, N- tetradecylidmethylbenzyl ammonium chloride monohydrate, dimethyl didecyl ammonium chloride, N-alkyl and (C₁₂₋₁₄) dimethyl 1-napthylmethyl ammonium chloride, trimethylammonium halide, alkyl-trimethylammonium salts, dialkyl- dimethylammonium salts, lauryl trimethyl ammonium chloride, ethoxylated alkyamidoalkyldialkylammonium salt, an ethoxylated trialkyl ammonium salt, dialkylbenzene dialkylammonium chloride, N-didecyldimethyl ammonium chloride, N-tetradecyldimethylbenzyl ammonium, chloride monohydrate, N-alkyl(C₁₂₋₁₄) dimethyl 1-naphthylmethyl ammonium chloride, dodecyldimethylbenzyl ammonium chloride, dialkyl benzenealkyl ammonium chloride, lauryl trimethyl ammonium chloride, alkylbenzyl methyl ammonium chloride, alkyl benzyl dimethyl ammonium bromide, C₁₂ trimethyl ammonium bromides, C₁₅ trimethyl ammonium bromides, C₁₇trimethyl ammonium bromides, dodecylbenzyl triethyl ammonium chloride, polydiallyldimethylammonium chloride (DADMAC), dimethyl ammonium chlorides, alkyldimethylammonium halogenides, tricetyl methyl ammonium chloride, decyltrimethylammonium bromide, dodecyltriethylammonium bromide, tetradecyltrimethylammonium bromide, methyl trioctylammonium chloride, tetrabutylammonium bromide, benzyl trimethylammonium bromide, choline esters, benzalkonium chloride, stearalkonium chloride compounds, cetyl pyridinium bromide, cetyl pyridinium chloride, halide salts of quaternized polyoxyethylalkylamines, alkyl pyridinium salts; amines, amine salts, amine oxides, imide azolinium salts, protonated quaternary acrylamides, methylated quaternary polymers, and cationic guar.

8. The composition of any of claims 1-6, wherein at least one surface stabilizer is selected from the group consisting of povidone, povidone polymer, hydroxypropyl cellulose, hydroxypropyl methyl cellulose, polyvinyl pyrrolidone, polyvinyl pyrrolidone polymers, high molecular weight polyoxyalkylene ethers, polyoxyethylene sorbitan fatty acid esters, polyethylene glycol phospholipids, dioxycholic acid, dioctylsulfosuccinate, sodium laurel sulphate, triblock copolymer surface modifiers, tyloxapol, and lecithin.

9. The composition of any preceding claim, additionally comprising one or more active agents useful for the treatment of mental diseases or disorders.

10. The composition of claim 9 wherein said mental disease or disorder is selected from the group consisting of schizophreniform illness, schizophrenia, bipolar disorder and combinations thereof.

11. The composition of claim 9 or 10, wherein said one or more active agents is selected from the group consisting of chlorpromazine, fluphenazine, perphenazine and prochlorperazine, clozapine, olanzapine, quetiapine, ziprasidone and combinations thereof.

12. A method of preparing a nanoparticulate aripiprazole, or a salt thereof, parenteral composition comprising: contacting particles of aripiprazole with at least one surface stabilizer for a time and under conditions sufficient to provide a nanoparticulate aripiprazole composition having an effective average particle size of less than about 2000 nm.

13. A composition according to any of claims 1-11, for use in the treatment of schizophrenia, bipolar disorder, schizophreniform illnesses and related conditions.

14. The composition of claim 13, further comprising one or more active agents useful for the treatment of schizophrenia, bipolar disorder and related condition selected from the group consisting of extrapyramidal symptoms, drug-induced Parkinsonism, acute dystonic reactions, akathisia, tardiv dyskinesia, tardive distonia and a combination thereof.
